# EUROPEAN PATENT SPECIFICATION

(11) **EP 1 876 226 B1**
(45) Date of publication and mention of the grant of the patent: **23.03.2011**
(21) Application number: 06116780.5
(22) Date of filing: 07.07.2006
(51) Int. Cl.: C12N 9/42

(54) **Detergent compositions**
Waschmittelzusammensetzungen
Compositions de lavage

(43) Date of publication of application: 09.01.2008
(73) Proprietor: The Procter & Gamble Company, Cincinnati, OH 45202 (US)
(72) Inventor: Lant, Neil, Newcastle upon Tyne, NE3 5RP (GB)
(74) Representative: Howard, Phillip Jan

(56) References cited:
- EP-A2- 0 265 832
- EP-A2- 0 271 004
- EP-A2- 1 350 843
- WO-A-02/061026
- WO-A-2006/055787
- WO-A2-02/099091
- WO-A2-2004/053039

## Description

### FIELD OF THE INVENTION

This invention relates to laundry detergent compositions comprising a bacterial alkaline enzyme exhibiting endo-beta-1,4-glucanase activity (E.C. 3.2.1.4) and a fabric hueing agent, and from 0.1% to 60% by weight of surfactant and processes for making and using such products.

### BACKGROUND OF THE INVENTION

Cellulase enzymes have been used in detergent compositions for many years now for their known benefits of depilling, softness and colour care. However, the use of most of cellulases has been limited because of the negative impact that cellulase may have on the tensile strength of the fabrics' fibers by hydrolysing crystalline cellulose. Recently, cellulases with a high specificity towards amorphous cellulose have been developed to exploit the cleaning potential of cellulases while avoiding the negative tensile strength loss. Especially alkaline endo-glucanases have been developed to suit better the use in alkaline detergent conditions.

For example, Novozymes in WO02/099091 discloses a novel enzyme exhibiting endo-beta-glucanase activity (EC 3.2.1.4) endogenous to the strain *Bacillus sp.,* DSM 12648; for use in detergent and textile applications. Novozymes further describes in WO04/053039 detergent compositions comprising an anti-redeposition endo-glucanase and its combination with certain cellulases having increased stability towards anionic surfactant and/or further specific enzymes. Kao's EP 265 832 describes novel alkaline cellulase K, CMCase I and CMCase II obtained by isolation from a culture product of *Bacillus sp* KSM-635. Kao further describes in EP 1 350 843, alkaline cellulase which acts favourably in an alkaline environment and can be mass produced readily because of having high secretion capacity or having enhanced specific activity.

WO 2004/053039, WO 2006/055787, WO 02/099091, WO 02/061206, EP1350843, EP0265832, and EP0271004 disclose enzymatic system and hueing agent.

We have found that the combination of alkaline bacterial endoglucanases and hueing agents deliver improved, synergistic whitening benefits. Without wishing to be bound by theory, it is believed that the following mechanisms are likely to give rise to such benefits: the endoglucanase enzyme hydrolyses amorphous cellulose present on the cotton surface, and thereby assists the removal of yellow soils and opens up the pore structure of the fabric making it more accessible to dye molecules. The resulting combination of improved yellow soil removal and improved shading colorant deposition leads to an improvement in fabric appearance. These combined effects hence contribute to a surprising improvement in visual perception and hence, in cleaning perception.

### SUMMARY OF THE INVENTION

The present invention relates to compositions comprising a fabric hueing agent and a bacterial alkaline enzyme exhibiting endo-beta-1,4-glucanase activity (E.C. 3.2.1.4), and from 0.1% to 60% by weight of surfactant.

### SEQUENCE LISTINGS

SEQ ID NO: 1 shows the amino acid sequence of an endoglucanase from *Bacillus sp.* AA349

SEQ ID NO: 2 shows the amino acid sequence of an endoglucanase from *Bacillus* sp KSM-S237

### DETAILED DESCRIPTION OF THE INVENTION

### DEFINITIONS

As used herein, the term "cleaning composition" includes, unless otherwise indicated, granular or powder-form all-purpose or "heavy-duty" washing agents, especially laundry detergents; liquid, gel or paste-form all-purpose washing agents, especially the so-called heavy-duty liquid types; liquid fine-fabric detergents; as well as cleaning auxiliaries such as bleach additives and "stain-stick" or pre-treat types.

As used herein the term 'fabric hueing agent' means dyes or pigments which when formulated in detergent compositions can deposit onto a fabric when said fabric is contacted with a wash liquor comprising said detergent compositions thus altering the tint of said fabric through absorption of visible light. For the purposes of the present application, fluorescent whitening agents, also called optical brighteners, are not considered fabric hueing agents, as they exert their effects on fabric through emission, rather than absorption, of visible light.

### COMPOSITIONS

The compositions of the present invention may contain from 0.00003% to 0.1%, from 0.00008% to 0.05%, or even from 0.0001% to 0.04% by weight of one or more fabric hueing agent and from 0.00005% to 0.15%, from 0.0002% to 0.02%, or even from 0.0005% to 0.01% by weight of pure enzyme, of one or more endoglucanase(s). The balance of any aspects of the aforementioned cleaning compositions is made up of one or more adjunct materials.

### SUITABLE ENDOGLUCANASE

The endoglucanase to be incorporated into the detergent composition of the present invention is one or more bacterial alkaline enzyme(s) exhibiting endo-beta-1,4-glucanase activity (E.C. 3.2.1.4).

As used herein, the term "alkaline endoglucanase", shall mean an endoglucanase having an optimum pH above 7 and retaining greater than 70% of its optimal activity at pH10.

Preferably, the endoglucanase is a bacterial polypeptide endogenous to a member of the genus *Bacillus.*
More preferably, the alkaline enzyme exhibiting endo-beta-1,4-glucanase activity (E.C. 3.2.1.4), is a polypeptide containing (i) at least one family 17 carbohydrate binding module (Family 17 CBM) and/or (ii) at least one family 28 carbohydrate binding module (Family 28 CBM). Please refer for example to: Current Opinion in Structural Biology, 2001, 593-600 by Y. Bourne and B. Henrissat in their article entitled: "Glycoside hydrolases and glycosyltransferases: families and functional modules" for the definition and classification of CBMs. Please refer further to Biochemical Journal, 2002, v361, 35-40 by A.B. Boraston et al in their article entitled: "Identification and glucan-binding properties of a new carbohydrate-binding module family" for the properties of the family 17 and 28 CBM's.

In a more preferred embodiment, said enzyme comprises a polypeptide (or variant thereof) endogenous to one of the following *Bacillus species:*

| **Bacillus sp.** | **As described in:** |
|---|---|
| AA349 (DSM 12648) | WO 2002/099091A (Novozymes) p2, line 25 |
| | WO 2004/053039A (Novozymes) p3, line19 |
| KSM S237 | EP 1350843A (Kao) p3, line 18 |
| 1139 | EP 1350843A (Kao) p3, line 22 |
| KSM 64 | EP 1350843A (Kao) p3, line 24 |
| KSM N 131 | EP 1350843A (Kao) p3, line 25 |
| KSM 635, FERM BP 1485 | EP 265 832A (Kao) p7, line 45 |
| KSM 534, FERM BP 1508 | EP 0271004 A (Kao) p9, line 21 |
| KSM 539, FERM BP 1509 | EP 0271004 A (Kao) p9, line 22 |
| KSM 577, FERM BP 1510 | EP 0271004 A (Kao) p9, line 22 |
| KSM 521, FERM BP 1507 | EP 0271004 (Kao) p9, line 19 |
| KSM 580, FERM BP 1511 | EP 0271004 (Kao) p9, line 20 |
| KSM 588, FERM BP 1513 | EP 0271004 A (Kao) p9, line 23 |
| KSM 597, FERM BP 1514 | EP 0271004 (Kao) p9, line 24 |
| KSM 522, FERM BP 1512 | EP 0271004 A (Kao) p9, line 20 |
| KSM 3445, FERM BP 1506 | EP 0271004 A (Kao) p10, line 3 |
| KSM 425. FERM BP 1505 | EP 0271004 (Kao) p10, line 3 |

Suitable endoglucanases for the compositions of the present invention are:
1) An enzyme exhibiting endo-beta-1,4-glucanase activity (E.C. 3.2.1.4), which has a sequence of at least 90%, preferably 94%, more preferably 97% and even more preferably 99%, 100% identity to the amino acid sequence of position 1 to position 773 of SEQ ID NO:1 (Corresponding to SEQ ID NO:2 in WO02/099091); or a fragment thereof that has endo-beta-1,4-glucanase activity, when identity is determined by GAP provided in the GCG program using a GAP creation penalty of 3.0 and GAP extension penalty of 0.1. The enzyme and the corresponding method of production is described extensively in patent application WO02/099091 published by Novozymes A/S on December 12, 2002. Please refer to the detailed description pages 4 to 17 and to the examples page 20 to page 26. One of such enzyme is commercially available under the tradename Celluclean™ by Novozymes A/S. GCG refers to the sequence analysis software package provided by Accelrys, San Diego, CA, USA. This incorporates a program called GAP which uses the algorithm of Needleman and Wunsch to find the alignment of two complete sequences that maximises the number of matches and minimises the number of gaps.
2) Also suitable are the alkaline endoglucanase enzymes described in EP 1 350 843A published by Kao corporation on October 8, 2003. Please refer to the detailed description [0011] to [0039] and examples 1 to 4 [0067] to [0077] for a detailed description of the enzymes and its production. The alkaline cellulase variants are obtained by substituting the amino acid residue of a cellulase having an amino acid sequence exhibiting at least 90%, preferably 95%, more preferably 98% and even 100% identity with the amino acid sequence represented by SEQ. ID NO:2 (Corresponding to SEQ. ID NO:1 in EP 1 350 843 on pages 11-13) at (a) position 10, (b) position 16, (c) position 22, (d) position 33, (e) position 39, (f) position 76, (g) position 109, (h) position 242, (i) position 263, (j) position 308, (k) position 462, (1) position 466, (m) position 468, (n) position 552, (o) position 564, or (p) position 608 in SEQ ID NO:2 or at a position corresponding thereto with another amino acid residue
Examples of the "alkaline cellulase having the amino acid sequence represented by SEQ. ID NO:2" include Eg1-237 [derived from *Bacillus* sp. strain KSM-S237 (FERM BP-7875), Hakamada, et al., Biosci. Biotechnol. Biochem., 64, 2281-2289, 2000]. Examples of the "alkaline cellulase having an amino acid sequence exhibiting at least 90% homology with the amino acid sequence represented by SEQ. ID NO:2" include alkaline cellulases having an amino acid sequence exhibiting preferably at least 95% homology, more preferably at least 98% homology, with the amino acid sequence represented by SEQ. ID NO:2. Specific examples include alkaline cellulase derived from *Bacillus* sp. strain 1139 (Egl-1139) (Fukumori, et al., J. Gen. Microbiol., 132, 2329-2335) (91.4% homology), alkaline cellulases derived from *Bacillus* sp. strain KSM-64 (Eg1-64) (Sumitomo, et al., Biosci. Biotechnol. Biochem., 56, 872-877, 1992) (homology: 91.9%), and cellulase derived from *Bacillus* sp. strain KSM-N13 1(Eg1-N131b) (Japanese Patent Application No. 2000-47237) (homology: 95.0%).
The amino acid is preferably substituted by: glutamine, alanine, proline or methionine, especially glutamine is preferred at position (a), asparagine or arginine, especially asparagine is preferred at position (b), proline is preferred at position (c), histidine is preferred at position (d), alanine, threonine or tyrosine, especially alanine is preferred at position (e), histidine, methionine, valine, threonine or alanine, especially histidine is preferred at position (f), isoleucine, leucine, serine or valine, especially isoleucine is preferred at position (g), alanine, phenylalanine, valine, serine, aspartic acid, glutamic acid, leucine, isoleucine, tyrosine, threonine, methionine or glycine, especially alanine, phenylalanine or serine is preferred at position (h), isoleucine, leucine, proline or valine, especially isoleucine is preferred at position (i), alanine, serine, glycine or valine, especially alanine is preferred at position (j), threonine, leucine, phenylalanine or arginine, especially threonine is preferred at position (k), leucine, alanine or serine, especially leucine is preferred at position (1), alanine, aspartic acid, glycine or lysine, especially alanine is preferred at position (m), methionine is preferred at position (n), valine, threonine or leucine, especially valine is preferred at position (o) and isoleucine or arginine, especially isoleucine is preferred at position (p).
The "amino acid residue at a position corresponding thereto" can be identified by comparing amino acid sequences by using known algorithm, for example, that of Lipman-Pearson's method, and giving a maximum similarity score to the multiple regions of simirality in the amino acid sequence of each alkaline cellulase. The position of the homologous amino acid residue in the sequence of each cellulase can be determined, irrespective of insertion or depletion existing in the amino acid sequence, by aligning the amino acid sequence of the cellulase in such manner (Fig. 1 of EP 1 350 843). It is presumed that the homologous position exists at the three-dimensionally same position and it brings about similar effects with regard to a specific function of the target cellulase.
With regard to another alkaline cellulase having an amino acid sequence exhibiting at least 90% homology with SEQ. ID NO:2, specific examples of the positions corresponding to (a) position 10, (b), position 16, (c) position 22, (d) position 33, (e) position 39, (f) position 76, (g) position 109, (h) position 242, (i) position 263, (j) position 308, (k) position 462, (l) position 466, (m) position 468, (n) position 552, (o) position 564 and (p) position 608 of the alkaline cellulase (Eg1-237) represented by SEQ. ID NO: 2 and amino acid residues at these positions will be shown below:

| | Egl-237 | Egl-1139 | Egl-64 | Egl-N131b |
|---|---|---|---|---|
| (a) | 10Leu | 10Leu | 10Leu | 10Leu |
| (b) | 16Ile | 16Ile | 16Ile | Nothing corresponding thereto |
| (c) | 22Ser | 22Ser | 22Ser | Nothing corresponding thereto |
| (d) | 33Asn | 33Asn | 33Asn | 19Asn |
| (e) | 39Phe | 39Phe | 39Phe | 25Phe |
| (f) | 76Ile | 76Ile | 76Ile | 62Ile |
| (g) | 109Met | 109Met | 109Met | 95Met |
| (h) | 242Gln | 242Gln | 242Gln | 228Gln |
| (i) | 263Phe | 263Phe | 263Phe | 249Phe |
| (j) | 308Thr | 308Thr | 308Thr | 294Thr |
| (k) | 462Asn | 461Asn | 461 Asn | 448Asn |
| (l) | 466Lys | 465Lys | 465Lys | 452Lys |
| (m) | 468Val | 467Val | 467Val | 454Val |
| (n) | 552Ile | 550Ile | 550Ile | 538Ile |
| (o) | 564Ile | 562Ile | 562Ile | 550Ile |
| (p) | 608Ser | 606Ser | 606Ser | 594Ser |

3) Also suitable is the alkaline cellulase K described in EP 265 832A published by Kao on May 4, 1988. Please refer to the description page 4, line 35 to page 12, line 22 and examples 1 and 2 on page 19 for a detailed description of the enzyme and its production. The alkaline cellulase K has the following physical and chemical properties:
- (1) Activity: Having a Cx enzymatic activity of acting on carboxymethyl cellulose along with a weak C₁ enzymatic activity and a weak beta-glucoxidase activity;
- (2) Specificity on Substrates: Acting on carboxymethyl cellulose(CMC), crystalline cellulose, Avicell, cellobiose, and p-nitrophenyl cellobioside(PNPC);
- (3) Having a working pH in the range of 4 to 12 and an optimum pH in the range of 9 to 10;
- (4) Having stable pH values of 4.5 to 10.5 and 6.8 to 10 when allowed to stand at 40°C for 10 minutes and 30 minutes, respectively;
- (5) Working in a wide temperature range of from 10 to 65°C with an optimum temperature being recognized at about 40°C;
- (6) Influences of chelating agents: The activity not impeded with ethylenediamine tetraacetic acid (EDTA), ethyleneglycol-bis-(β-aminoethylether) N,N,N',N"-tetraacetic acid (EGTA), N,N-bis(carboxymethyl)glycine (nitrilotriacetic acid) (NTA), sodium tripolyphosphate (STPP) and zeolite;
- (7) Influences of surface active agents: Undergoing little inhibition of activity by means of surface active agents such as sodium linear alkylbenzenesulfonates (LAS), sodium alkylsulfates (AS), sodium polyoxyethylene alkylsulfates (ES), sodium alphaolefinsulfonates (AOS), sodium alpha-sulfonated aliphatic acid esters (alpha-SFE), sodium alkylsulfonates (SAS), polyoxyethylene secondary alkyl ethers, fatty acid salts (sodium salts), and dimethyldialkylammonium chloride;
- (8) Having a strong resistance to proteinases; and
- (9) Molecular weight (determined by gel chromatography): Having a maximum peak at 180,000 ± 10,000.

Preferably such enzyme is obtained by isolation from a culture product of *Bacillus sp* KSM-635.
Cellulase K is commercially available by the Kao Corporation: e.g. the cellulase preparation Eg-X known as KAC® being a mixture of E-H and E-L both from Bacillus sp. KSM-635 bacterium. Cellulases E-H and E-L have been described in S. Ito, Extremophiles, 1997, v1, 61-66 and in S. Ito et al, Agric Biol Chem, 1989, v53, 1275-1278.
4) The alkaline bacterial endoglucanases described in EP 271 004A published by Kao on June 15, 1988 are also suitable for the purpose of the present invention. Please refer to the description page 9, line 15 to page 23, line 17 and page 31, line 1 to page 33, line 17 for a detailed description of the enzymes and its production. Those are:
Alkaline Cellulase K-534 from KSM 534, FERM BP 1508,
Alkaline Cellulase K-539 from KSM 539, FERM BP 1509,
Alkaline Cellulase K-577 from KSM 577, FERM BP 1510,
Alkaline Cellulase K-521 from KSM 521, FERM BP 1507,
Alkaline Cellulase K-580 from KSM 580, FERM BP 1511,
Alkaline Cellulase K-588 from KSM 588, FERM BP 1513,
Alkaline Cellulase K-597 from KSM 597, FERM BP 1514,
Alkaline Cellulase K-522 from KSM 522, FERM BP 1512,
Alkaline Cellulase E-II from KSM 522, FERM BP 1512,
Alkaline Cellulase E-III from KSM 522, FERM BP 1512.
Alkaline Cellulase K-344 from KSM 344, FERM BP 1506, and
Alkaline Cellulase K-425 from KSM 425, FERM BP 1505.

5) Finally, the alkaline endoglucanases derived from Bacillus species KSM-N described in JP2005287441A, published by Kao on the October 20^{th}, 2005, are also suitable for the purpose of the present invention. Please refer to the description page 4, line 39 to page 10, line 14 for a detailed description of the enzymes and its production. Examples of such alkaline endoglucanases are:
Alkaline Cellulase Egl-546H from Bacillus sp. KSM-N546
Alkaline Cellulase Egl-115 from Bacillus sp. KSM-N115
Alkaline Cellulase Egl-145 from Bacillus sp. KSM-N145
Alkaline Cellulase Egl-659 from Bacillus sp.KSM-N659
Alkaline Cellulase Egl-640 from Bacillus sp.KSM-N440

Also encompassed in the present invention are variants of the above described enzymes obtained by various techniques known by persons skilled in the art such as directed evolution.

### FABRIC HUEING AGENTS

Fluorescent whitening agents emit at least some visible light. In contrast, fabric hueing agents can alter the tint of a surface as they absorb at least a portion of the visible light spectrum. Suitable fabric hueing agents include dyes and dye-clay conjugates, and may also include pigments. In one aspect, suitable fabric hueing agents include those fabric hueing agents that satisfy the requirements of Test Method 1 in the Test Method Section of the present specification. Suitable dyes include small molecule dyes and polymeric dyes.

Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example:
(1) Tris-azo direct blue dyes of the formula 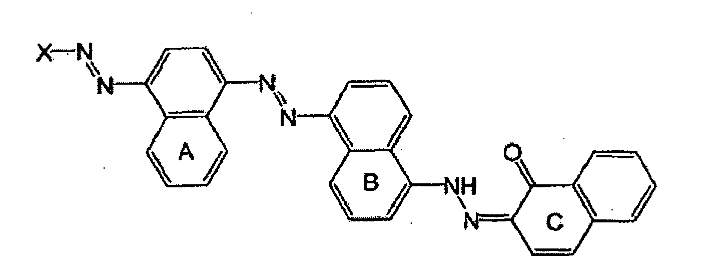 where at least two of the A, B and C napthyl rings are substituted by a sulfonate group, the C ring may be substituted at the 5 position by an NH₂ or NHPh group, X is a benzyl or naphthyl ring substituted with up to 2 sulfonate groups and may be substituted at the 2 position with an OH group and may also be substituted with an NH₂ or NHPh group.
(2) bis-azo Direct violet dyes of the formula: 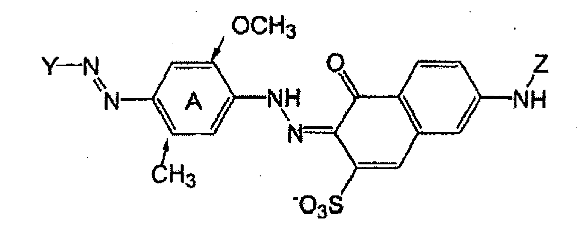 where Z is H or phenyl, the A ring is preferably substituted by a methyl and methoxy group at the positions indicated by arrows, the A ring may also be a naphthyl ring, the Y group is a benzyl or naphthyl ring, which is substituted by sulfate group and may be mono or disubstituted by methyl groups.
(3) Blue or red acid dyes of the formula 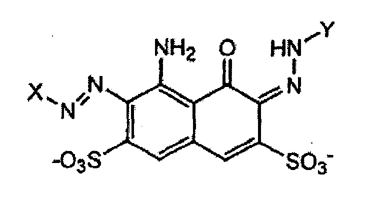 where at least one of X and Y must be an aromatic group. In one aspect, both the aromatic groups may be a substituted benzyl or naphthyl group, which may be substituted with non water-solubilising groups such as alkyl or alkyloxy or aryloxy groups, X and Y may not be substituted with water solubilising groups such as sulfonates or carboxylates. In another aspect, X is a nitro substituted benzyl group and Y is a benzyl group
(4) Red acid dyes of the structure 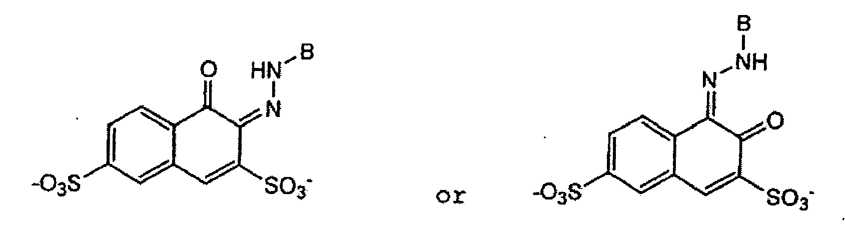 where B is a naphthyl or benzyl group that may be substituted with non water solubilising groups such as alkyl or alkyloxy or aryloxy groups, B may not be substituted with water solubilising groups such as sulfonates or carboxylates.
(5) Dis-azo dyes of the structure 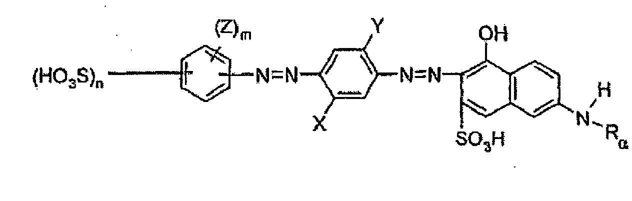 or 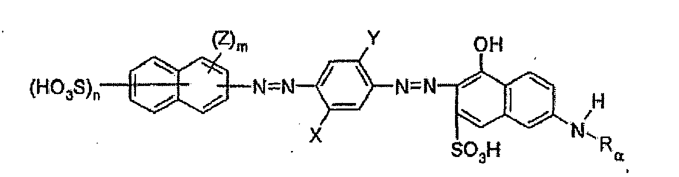 wherein X and Y, independently of one another, are each hydrogen, C₁-C₄ alkyl or C₁-C₄-alkoxy, Rα is hydrogen or aryl, Z is C₁-C₄ alkyl; C₁-C₄-alkoxy; halogen; hydroxyl or carboxyl, n is 1 or 2 and m is 0, 1 or 2, as well as corresponding salts thereof and mixtures thereof
(6) Triphenylmethane dyes of the following structures 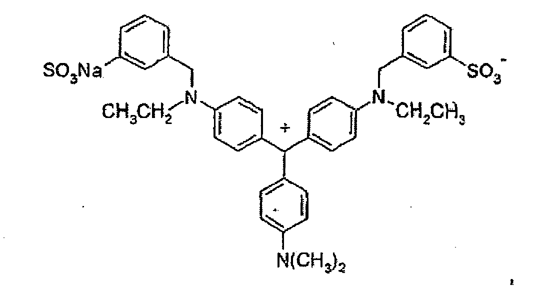 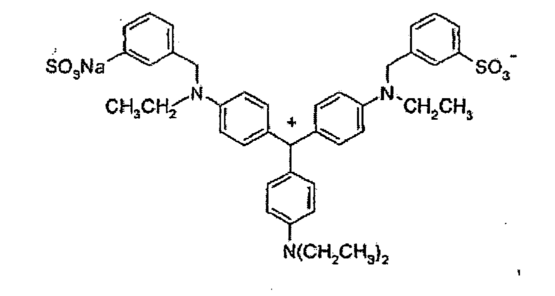 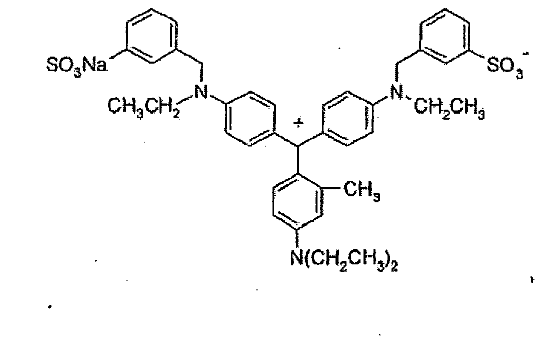 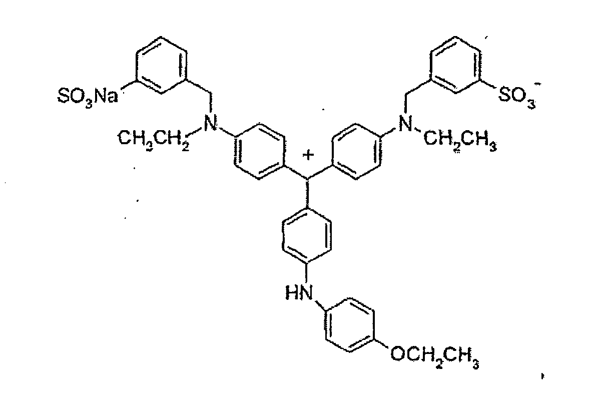 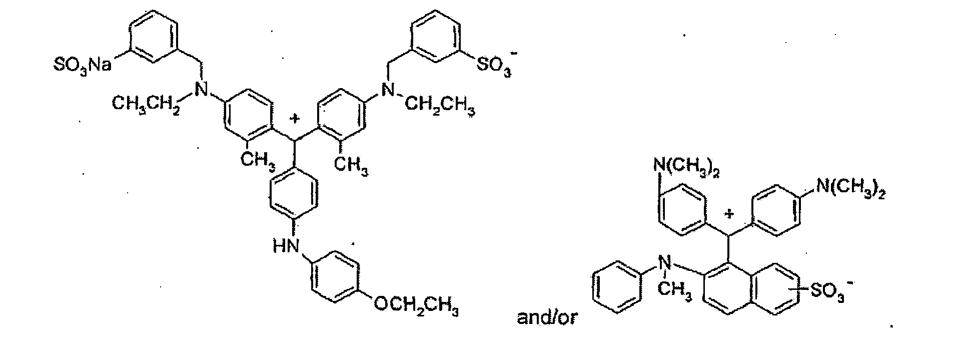
and mixtures thereof.

In another aspect, suitable small molecule dyes include small molecule dyes selected from the group consisting of Colour Index (Society of Dyers and Colourists, Bradford, UK) numbers Direct Violet 9, Direct Violet 35, Direct Violet 48, Direct Violet 51, Direct Violet 66, Direct Blue 1, Direct Blue 71, Direct Blue 80, Direct Blue 279, Acid Red 17, Acid Red 73, Acid Red 88, Acid Red 150, Acid Violet 15, Acid Violet 17, Acid Violet 24, Acid Violet 43, Acid Violet 49, Acid Blue 15, Acid Blue 17, Acid Blue 25, Acid Blue 29, Acid Blue 40, Acid Blue 45, Acid Blue 75, Acid Blue 80, Acid Blue 83, Acid Blue 90 and Acid Blue 113, Acid Black 1, Basic Violet 1, Basic Violet 3, Basic Violet 4, Basic Violet 10, Basic Violet 35, Basic Blue 3, Basic Blue 16, Basic Blue 22, Basic Blue 47, Basic Blue 66, Basic Blue 75, Basic Blue 159 and mixtures thereof. In another aspect, suitable small molecule dyes include small molecule dyes selected from the group consisting of Colour Index (Society of Dyers and Colourists, Bradford, UK) numbers Acid Violet 17, Acid Violet 43, Acid Red 73, Acid Red 88, Acid Red 150, Acid Blue 25, Acid Blue 29, Acid Blue 45, Acid Blue 113, Acid Black 1, Direct Blue 1, Direct Blue 71, Direct Violet 51 and mixtures thereof.

In another aspect, suitable small molecule dyes include small molecule dyes selected from the group consisting of Colour Index (Society of Dyers and Colourists, Bradford, UK) numbers Acid Violet 17, Direct Blue 71, Direct Violet 51, Direct Blue 1, Acid Red 88, Acid Red 150, Acid Blue 29, Acid Blue 113 or mixtures thereof.

In another aspect, suitable small molecule dyes include photobleaches which satisfy the requirements of Test Method 1 in the Test Method Section. Such materials function as both fabric hueing dyes and also as photobleaching agents, i.e. they generate bleaching species on exposure to light. Suitable photobleaches include catalytic photobleaches selected from the group consisting of water soluble phthalocyanines of the formula: 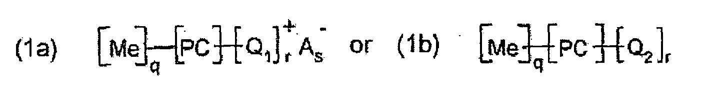 in which:
- PC: is the phthalocyanine ring system;
- Me: is Zn; Fe(II); Ca; Mg; Na; K; Al-Z₁; Si(IV) ; P(V); Ti(IV); Ge(IV); Cr(VI); Ga(III); Zr(IV); In(III); Sn(IV) or Hf(VI) ;
- Z₁: is a halide; sulfate; nitrate; carboxylate; alkanolate; or hydroxyl ion;
- q: is 0; 1 or 2;
- r: is 1 to 4;
- Q₁,: is a sulfo or carboxyl group; or a radical of the formula -SO₂X₂-R₁-
- X₃⁺; - R₁ or: O-R₁-X₃⁺; or -(CH₂),-Y₁⁺; in which
R₁ is a branched or unbranched C₁-C₈ alkylene; or 1,3- 1,4-phenylene;
X₂ is -NH-; or -N-C₁-C₅ alkyl;
X₃⁺ is a group of the formula 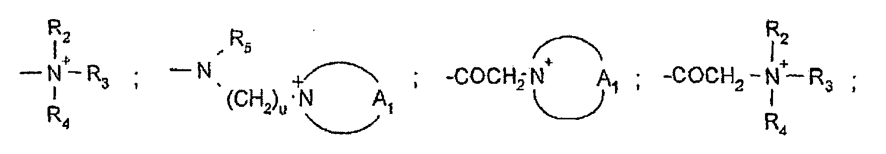
or, in the case where R, =C₁-C₈alkylene, also a group of the formula 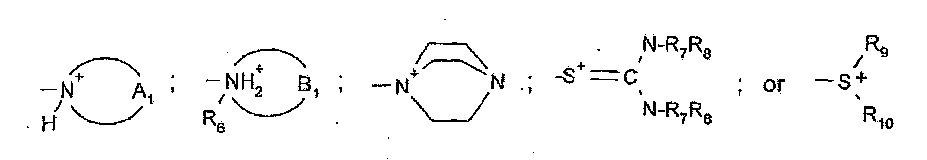 Y₁⁺ is a group of the formula 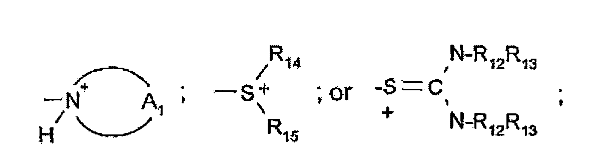 t is 0 or 1
where in the above formulae
R₂ and R₃ independently of one another are C₁-C₆ alkyl
R₄ is C₁-C₅ alkyl; C₅-C₇ cycloalkyl or NR₇R₈;
R₅ and R₆ independently of one another are C₁-C₅ alkyl;
R₇ and R₈ independently of one another are hydrogen or C₁-C₅ alkyl;
R₉ and R₁₀ independently of one another are unsubstituted C₁-C₆ alkyl or C₁-C₆ alkyl substituted by hydroxyl, cyano, carboxyl, carb-C₁-C₆ alkoxy, C₁-C₆ alkoxy, phenyl, naphthyl or pyridyl;
u is from 1 to 6;
A₁ is a unit which completes an aromatic 5- to 7-membered nitrogen heterocycle, which may where appropriate also contain one or two further nitrogen atoms as ring members, and
B₁ is a unit which completes a saturated 5- to 7-membered nitrogen heterocycle, which may where appropriate also contain 1 to 2 nitrogen, oxygen and/or sulfur atoms as ring members;
Q₂ is hydroxyl; C₁-C₂₂ alkyl; branched C₃-C₂₂ alkyl; C₂-C₂₂ alkenyl; branched C₃-C₂₂ alkenyl and mixtures thereof; C₁-C₂₂ alkoxy; a sulfo or carboxyl radical; a radical of the formula 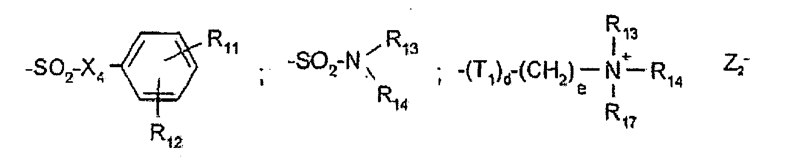 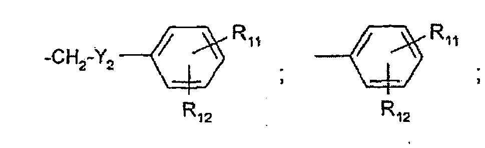 -SO₂(CH₂)ᵥ-OSO₃M; -SO₂(CH₂)ᵥ-SO₃M; 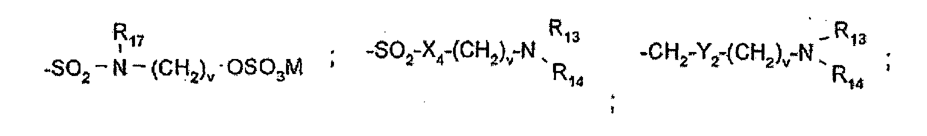 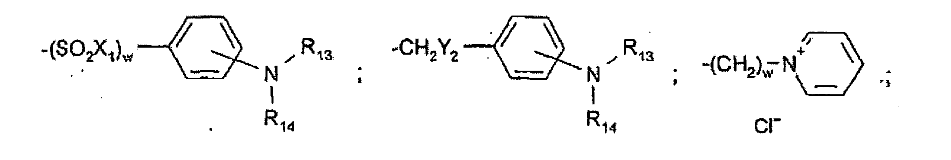 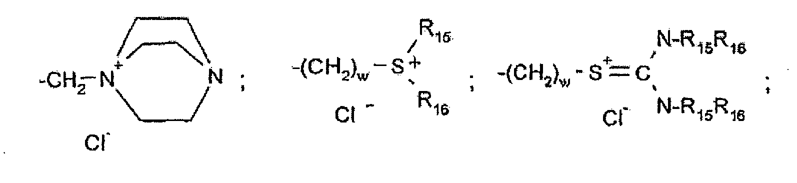 a branched alkoxy radical of the formula 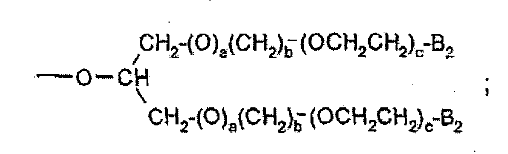 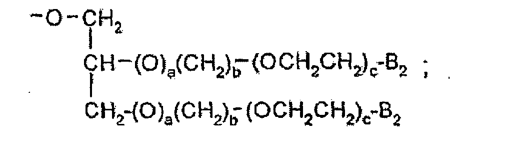 an alkylethyleneoxy unit of the formula

-(T₁)_{d}-(CH₂)_{b}(OCH₂CH₂)ₐ-B₃

or an ester of the formula

COOR₁₈

in which
B₂ is hydrogen; hydroxyl; C₁-C₃₀ alkyl; C₁-C₃₀ alkoxy; -CO₂H; -CH₂COOH; -SO₃-M₁; - OSO₃-M₁; -PO₃²⁻M₁; -OPO₃²⁻M₁; and mixtures thereof;
B₃ is hydrogen; hydroxyl; -COOH; -SO₃-M₁; -OSO₃ M₁ or C₁-C₆ alkoxy;
M₁ is a water-soluble cation;
T₁ is-O-; or-NH-;
X₁ and X₄ independently of one another are -O-; -NH- or -N-C₁-C₅alkyl;
R₁₁ and R₁₂ independently of one another are hydrogen; a sulfo group and salts thereof; a carboxyl group and salts thereof or a hydroxyl group; at least one of the radicals R₁₁ and R₁₂ being a sulfo or carboxyl group or salts thereof,
Y₂ is -O-; -S-; -NH- or -N-C₁-C₅alkyl;
R₁₃ and R₁₄ independently of one another are hydrogen; C₁-C₆ allcyl; hydroxy-C₁-C₆ alkyl; cyano-C₁-C₆ alkyl; sulfo- C₁-C₆ alkyl; carboxy or halogen-C₁-C₆ alkyl; unsubstituted phenyl or phenyl substituted by halogen, C₁-C₄ alkyl or C₁-C₄ alkoxy; sulfo or carboxyl or R₁₃ and R₁₄ together with the nitrogen atom to which they are bonded form a saturated 5- or 6- membered heterocyclic ring which may additionally also contain a nitrogen or oxygen atom as a ring member;
R₁₅ and R₁₆ independently of one another are C₁-C₆ alkyl or aryl-C₁-C₆ alkyl radicals;
R₁₇ is hydrogen; an unsubstituted C₁-C₆ alkyl or C₁-C₆ alkyl substituted by halogen, hydroxyl, cyano, phenyl, carboxyl, carb-C₁-C₆ alkoxy or C₁-C₆ alkoxy;
R₁₈ is C₁- C₂₂ alkyl; branched C₃-C₂₂ alkyl; C₁-C₂₂ alkenyl or branched C₃- C₂₂ alkenyl; C₃-C₂₂ glycol; C₁-C₂₂ alkoxy; branched C₃-C₂₂ alkoxy; and mixtures thereof;
M is hydrogen; or an alkali metal ion or ammonium ion,
Z₂⁻ is a chlorine; bromine; alkylsulfate or arylsulfate ion;
a is 0 or 1 ;
b is from 0 to 6;
c is from 0 to 100;
d is 0; or 1;
e is from 0 to 22;
v is an integer from 2 to 12;
w is 0 or 1; and
A⁻ is an organic or inorganic anion, and
s is equal to r in cases of monovalent anions A⁻ and less than or equal to r in cases of polyvalent anions, it being necessary for Aₛ⁻ to compensate the positive charge; where, when r is not equal to 1, the radicals Q₁ can be identical or different,
and where the phthalocyanine ring system may also comprise further solubilising groups;

Other suitable catalytic photobleaches include (i) xanthene dyes and mixtures thereof; and (ii) those selected from the group consisting of sulfonated zinc phthalocyanine, sulfonated aluminium phthalocyanine, Eosin Y, Phoxine B, Rose Bengal, C.I. Food Red 14 and mixtures thereof.

In another embodiment, fabric hueing dyes include photobleach-dye conjugates. Such materials contain at least one chromogen and at least one photobleach moiety in the same molecule. These include materials comprising at least one Zn-, Ca-, Mg-, Na-, K-, Al-, Si-, Ti-, Ge-, Ga-, Zr-, In- or Sn-phthalocyanine to which at least one dyestuff is attached through a covalent bonding. Examples of suitable materials are given below.

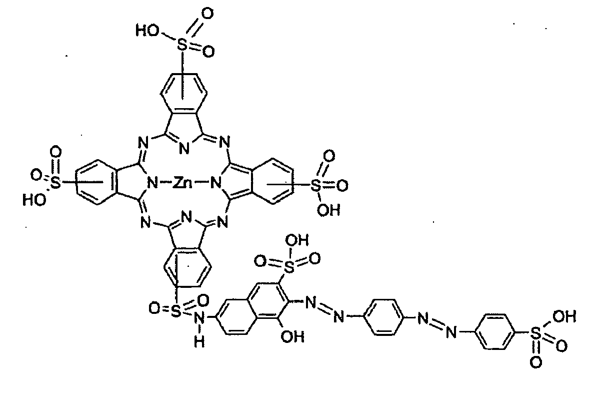

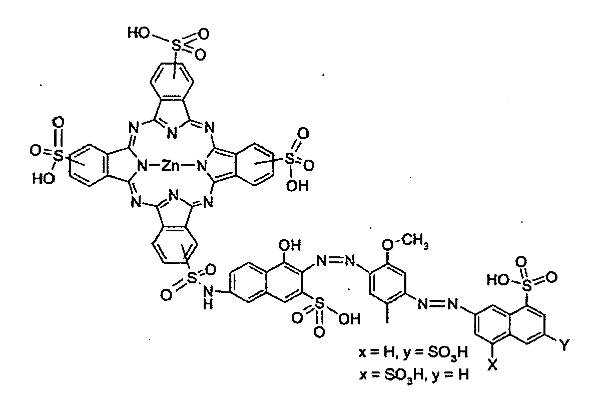

Suitable polymeric dyes include polymeric dyes selected from the group consisting of polymers containing conjugated chromogens (dye-polymer conjugates) and polymers with chromogens co-polymerised into the backbone of the polymer and mixtures thereof.
In another aspect, suitable polymeric dyes include polymeric dyes selected from the group consisting of fabric-substantive colorants sold under the name of Liquitint® (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. In still another aspect, suitable polymeric dyes include polymeric dyes selected from the group consisting of Liquitint® (Milliken, Spartanburg, South Carolina, USA) Violet CT, carboxymethyl cellulose (CMC) conjugated with a reactive blue, reactive violet or reactive red dye such as CMC conjugated with C.I. Reactive Blue 19, sold by Megazyme, Wicklow, Ireland under the product name AZO-CM-CELLULOSE, product code S-ACMC and mixtures thereof.

Suitable dye clay conjugates include dye clay conjugates selected from the group comprising at least one cationic/basic dye and a smectite clay, and mixtures thereof. In another aspect, suitable dye clay conjugates include dye clay conjugates selected from the group consisting of one cationic/basic dye selected from the group consisting of C.I. Basic Yellow 1 through 108, C.I. Basic Orange 1 through 69, C.I. Basic Red 1 through 118, C.I. Basic Violet 1 through 51, C.I. Basic Blue 1 through 164, C.I. Basic Green 1 through 14, C.I. Basic Brown 1 through 23, CI Basic Black 1 through 11, and a clay selected from the group consisting of Montmorillonite clay, Hectorite clay, Saponite clay and mixtures thereof. In still another aspect, suitable dye clay conjugates include dye clay conjugates selected from the group consisting of Montmorillonite Basic Blue B7 C.I. 42595 conjugate, Montmorillonite Basic Blue B9 C.I. 52015 conjugate, Montmorillonite Basic Violet V3 C.I. 42555 conjugate, Montmorillonite Basic Green G1 C.I. 42040 conjugate, Montmorillonite Basic Red R1 C.I. 45160 conjugate, Montmorillonite C.I. Basic Black 2 conjugate, Hectorite Basic Blue B7 C.I. 42595 conjugate, Hectorite Basic Blue B9 C.I. 52015 conjugate, Hectorite Basic Violet V3 C.I. 42555 conjugate, Hectorite Basic Green G1 C.I. 42040 conjugate, Hectorite Basic Red R1 C.I. 45160 conjugate, Hectorite C.I. Basic Black 2 conjugate, Saponite Basic Blue B7 C.I. 42595 conjugate, Saponite Basic Blue B9 C.I. 52015 conjugate, Saponite Basic Violet V3 C.I. 42555 conjugate, Saponite Basic Green G1 C.I. 42040 conjugate, Saponite Basic Red R1 C.I. 45160 conjugate, Saponite C.I. Basic Black 2 conjugate and mixtures thereof.

Suitable pigments include pigments selected from the group consisting of flavanthrone, indanthrone, chlorinated indanthrone containing from 1 to 4 chlorine atoms, pyranthrone, dichloropyranthrone, monobromodichloropyranthrone, dibromodichloropyranthrone, tetrabromopyranthrone, perylene-3,4,9,10-tetracarboxylic acid diimide, wherein the imide groups may be unsubstituted or substituted by C1-C3 - alkyl or a phenyl or heterocyclic radical, and wherein the phenyl and heterocyclic radicals may additionally carry substituents which do not confer solubility in water, anthrapyrimidinecarboxylic acid amides, violanthrone, isoviolanthrone, dioxazine pigments, copper phthalocyanine which may contain up to 2 chlorine atoms per molecule, polychloro-copper phthalocyanine or polybromochloro-copper phthalocyanine containing up to 14 bromine atoms per molecule and mixtures thereof.
In another aspect, suitable pigments include pigments selected from the group consisting of Ultramarine Blue (C.I. Pigment Blue 29), Ultramarine Violet (C.I. Pigment Violet 15) and mixtures thereof. In one aspect, Applicant's invention does not include Ultramarine Blue.

The aforementioned fabric hueing agents can be used in combination (any mixture of fabric hueing agents can be used). Suitable fabric hueing agents can be purchased from Aldrich, Milwaukee, Wisconsin, USA; Ciba Specialty Chemicals, Basel, Switzerland; BASF, Ludwigshafen, Germany; Dayglo Color Corporation, Mumbai, India; Organic Dyestuffs Corp., East Providence, Rhode Island, USA; Dystar, Frankfurt, Germany; Lanxess, Leverkusen, Germany; Megazyme, Wicklow, Ireland; Clariant, Muttenz, Switzerland; Avecia, Manchester, UK and/or made in accordance with the examples contained herein.

### Adjunct Materials

While not essential for the purposes of the present invention, the non-limiting list of adjuncts illustrated hereinafter are suitable for use in the instant compositions and may be desirably incorporated in certain embodiments of the invention, for example to assist or enhance cleaning performance, for treatment of the substrate to be cleaned, or to modify the aesthetics of the cleaning composition as is the case with perfumes, colorants, dyes or the like. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, dispersants, additional enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids, solvents and/or pigments. In addition to the disclosure below, suitable examples of such other adjuncts and levels of use are found in U.S. Patent Nos. 5,576,282, 6,306,812 B1 and 6,326,348 B1 . When one or more adjuncts are present, such one or more adjuncts may be present as detailed below:
Bleaching Agents - The cleaning compositions of the present invention may comprise one or more bleaching agents. Suitable bleaching agents other than bleaching catalysts include other photobleaches, bleach activators, hydrogen peroxide, sources of hydrogen peroxide, pre-formed peracids and mixtures thereof. In general, when a bleaching agent is used, the compositions of the present invention may comprise from about 0.1% to about 50% or even from about 0.1% to about 25% bleaching agent by weight of the subject cleaning composition. Examples of suitable bleaching agents include:
   (1) other photobleaches for example Vitamin K3;
   (2) preformed peracids: Suitable preformed peracids include, but are not limited to, compounds selected from the group consisting of percarboxylic acids and salts, percarbonic acids and salts, perimidic acids and salts, peroxymonosulfuric acids and salts, for example, Oxone ®, and mixtures thereof. Suitable percarboxylic acids include hydrophobic and hydrophilic peracids having the formula R-(C=O)O-O-M wherein R is an alkyl group, optionally branched, having, when the peracid is hydrophobic, from 6 to 14 carbon atoms, or from 8 to 12 carbon atoms and, when the peracid is hydrophilic, less than 6 carbon atoms or even less than 4 carbon atoms; and M is a counterion, for example, sodium, potassium or hydrogen;
   (3) sources of hydrogen peroxide, for example, inorganic perhydrate salts, including alkali metal salts such as sodium salts of perborate (usually mono- or tetra-hydrate), percarbonate, persulphate, perphosphate, persilicate salts and mixtures thereof. In one aspect of the invention the inorganic perhydrate salts are selected from the group consisting of sodium salts of perborate, percarbonate and mixtures thereof. When employed, inorganic perhydrate salts are typically present in amounts of from 0.05 to 40 wt%, or 1 to 30 wt% of the overall composition and are typically incorporated into such compositions as a crystalline solid that may be coated. Suitable coatings include, inorganic salts such as alkali metal silicate, carbonate or borate salts or mixtures thereof, or organic materials such as water-soluble or dispersible polymers, waxes, oils or fatty soaps; and
   (4) bleach activators having R-(C=O)-L wherein R is an alkyl group, optionally branched, having, when the bleach activator is hydrophobic, from 6 to 14 carbon atoms, or from 8 to 12 carbon atoms and, when the bleach activator is hydrophilic, less than 6 carbon atoms or even less than 4 carbon atoms; and L is leaving group. Examples of suitable leaving groups are benzoic acid and derivatives thereof - especially benzene sulphonate. Suitable bleach activators include dodecanoyl oxybenzene sulphonate, decanoyl oxybenzene sulphonate, decanoyl oxybenzoic acid or salts thereof, 3,5,5-trimethyl hexanoyloxybenzene sulphonate, tetraacetyl ethylene diamine (TAED) and nonanoyloxybenzene sulphonate (NOBS). Suitable bleach activators are also disclosed in WO 98/17767. While any suitable bleach activator may be employed, in one aspect of the invention the subject cleaning composition may comprise NOBS, TAED or mixtures thereof.

   When present, the peracid and/or bleach activator is generally present in the composition in an amount of from about 0.1 to about 60 wt%, from about 0.5 to about 40 wt % or even from about 0.6 to about 10 wt% based on the composition. One or more hydrophobic peracids or precursors thereof may be used in combination with one or more hydrophilic peracid or precursor thereof.
   The amounts of hydrogen peroxide source and peracid or bleach activator may be selected such that the molar ratio of available oxygen (from the peroxide source) to peracid is from 1:1 to 35:1, or even 2:1 to 10:1.
Surfactants - The cleaning compositions according to the present invention may comprise a surfactant or surfactant system wherein the surfactant can be selected from nonionic surfactants, anionic surfactants, cationic surfactants, ampholytic surfactants, zwitterionic surfactants, semi-polar nonionic surfactants and mixtures thereof. When present, surfactant is typically present at a level of from about 0.1% to about 60%, from about 1% to about 50% or even from about 5% to about 40% by weight of the subject composition.
Builders - The cleaning compositions of the present invention may comprise one or more detergent builders or builder systems. When a builder is used, the subject composition will typically comprise at least about 1%, from about 5% to about 60% or even from about 10% to about 40% builder by weight of the subject composition.
   Builders include, but are not limited to, the alkali metal, ammonium and alkanolammonium salts of polyphosphates, alkali metal silicates, alkaline earth and alkali metal carbonates, aluminosilicate builders and polycarboxylate compounds, ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof.
Chelating Agents - The cleaning compositions herein may contain a chelating agent. Suitable chelating agents include copper, iron and/or manganese chelating agents and mixtures thereof When a chelating agent is used, the subject composition may comprise from about 0.005% to about 15% or even from about 3.0% to about 10% chelating agent by weight of the subject composition.
   Dye Transfer Inhibiting Agents - The cleaning compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001% to about 10%, from about 0.01% to about 5% or even from about 0.1 % to about 3% by weight of the composition.
Fluorescent whitening agent - The cleaning compositions of the present invention will preferably also contain additional components that may tint articles being cleaned, such as fluorescent whitening agent. Any fluorescent whitening agent suitable for use in a laundry detergent composition may be used in the composition of the present invention. The most commonly used fluorescent whitening agents are those belonging to the classes of diaminostilbene-sulphonic acid derivatives, diarylpyrazoline derivatives and bisphenyl-distyryl derivatives. Examples of the diaminostilbene-sulphonic acid derivative type of fluorescent whitening agents include the sodium salts of:
   4,4'-bis-(2-diethanolamino-4-anilino-s-triazin-6-ylamino) stilbene-2,2'-disulphonate,
   4,4'-bis-(2,4-dianilino-s-triazin-6-ylamino) stilbene-2.2'-disulphonate,
   4,4'-bis-(2-anilino-4(N-methyl-N-2-hydroxy-ethylamino)-s-triazin-6-ylamino) stilbene-2,2'-disulphonate,
   4,4'-bis-(4-phenyl-2,1,3-triazol-2-yl)stilbene-2,2'-disulphonate,
   4,4'-bis-(2-anilino-4(1-methyl-2-hydroxy-ethylamino)-s-triazin-6-ylamino) stilbene-2,2'-disulphonate and,
   2-(stilbyl-4"-naptho-1.,2':4,5)-1,2,3-trizole-2"-sulphonate.

   Preferred fluorescent whitening agents are Tinopal® DMS and Tinopal® CBS available from Ciba-Geigy AG, Basel, Switzerland. Tinopal® DMS is the disodium salt of 4,4'-bis-(2-morpholino-4 anilino-s-triazin-6-ylamino) stilbene disulphonate. Tinopal® CBS is the disodium salt of 2,2'-bis-(phenyl-styryl) disulphonate.
   Also preferred are fluorescent whitening agents of the structure: 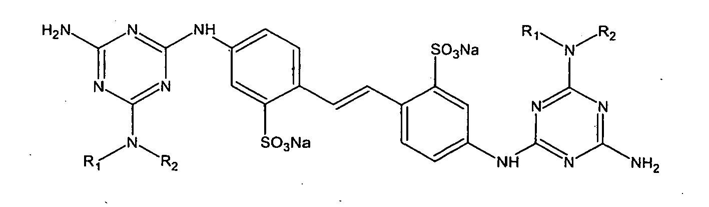 wherein R1 and R2, together with the nitrogen atom linking them, form an unsubstituted or C1-C4 alkyl- substituted morpholino, piperidine or pyrrolidine ring, preferably a morpholino ring (commercially available as Parawhite KX, supplied by Paramount Minerals and Chemicals, Mumbai, India)
   Other fluorescers suitable for use in the invention include the 1-3-diaryl pyrazolines and the 7-alkylaminocoumarins.
   Suitable fluorescent brightener levels include lower levels of from about 0.01, from about 0.05, from about 0.1 or even from about 0.2 wt % to upper levels of 0.5 or even 0.75 wt %.
Dispersants - The compositions of the present invention can also contain dispersants. Suitable water-soluble organic materials include the homo- or co-polymeric acids or their salts, in which the polycarboxylic acid comprises at least two carboxyl radicals separated from each other by not more than two carbon atoms.
Enzymes - In addition to the bacterial alkaline endoglucanase, the cleaning compositions can comprise one or more other enzymes which provide cleaning performance and/or fabric care benefits. Examples of suitable enzymes include, but are not limited to, hemicellulases, peroxidases, proteases, other cellulases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof. In a preferred embodiment, the compositions of the present invention will further comprise a lipase, for further improved cleaning and whitening performance. A typical combination is an enzyme cocktail that may comprise, for example, a protease and lipase in conjunction with amylase. When present in a cleaning composition, the aforementioned additional enzymes may be present at levels from about 0.00001 % to about 2%, from about 0.0001 % to about 1% or even from about 0.001 % to about 0.5% enzyme protein by weight of the composition.
Enzyme Stabilizers - Enzymes for use in detergents can be stabilized by various techniques. The enzymes employed herein can be stabilized by the presence of water-soluble sources of calcium and/or magnesium ions in the finished compositions that provide such ions to the enzymes. In case of aqueous compositions comprising protease, a reversible protease inhibitor, such as a boron compound, can be added to further improve stability.
Catalytic Metal Complexes - Applicants' cleaning compositions may include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra(methylenephosphonic acid) and water-soluble salts thereof. Such catalysts are disclosed in U.S. 4,430,243.
   If desired, the compositions herein can be catalyzed by means of a manganese compound. Such compounds and levels of use are well known in the art and include, for example, the manganese-based catalysts disclosed in U.S. 5,576,282.
   Cobalt bleach catalysts useful herein are known, and are described, for example, in U.S. 5,597,936; U.S. 5,595,967. Such cobalt catalysts are readily prepared by known procedures, such as taught for example in U.S. 5,597,936, and U.S. 5,595,967. Compositions herein may also suitably include a transition metal complex of ligands such as bispidones (WO 05/042532 A1) and/or macropolycyclic rigid ligands - abbreviated as "MRLs". As a practical matter, and not by way of limitation, the compositions and processes herein can be adjusted to provide on the order of at least one part per hundred million of the active MRL species in the aqueous washing medium, and will typically provide from about 0.005 ppm to about 25 ppm, from about 0.05 ppm to about 10 ppm, or even from about 0. 1 ppm to about 5 ppm, of the MRL in the wash liquor.
   Suitable transition-metals in the instant transition-metal bleach catalyst include, for example, manganese, iron and chromium. Suitable MRLs include 5,12-diethyl-1,5,8,12-tetraazabicyclo[6.6.2]hexadecane.
   Suitable transition metal MRLs are readily prepared by known procedures, such as taught for example in WO 00/32601, and U.S. 6,225,464.
Solvents - Suitable solvents include water and other solvents such as lipophilic fluids. Examples of suitable lipophilic fluids include siloxanes, other silicones, hydrocarbons, glycol ethers, glycerine derivatives such as glycerine ethers, perfluorinated amines, perfluorinated and hydrofluoroether solvents, low-volatility nonfluorinated organic solvents, diol solvents, other environmentally-friendly solvents and mixtures thereof.
Softening system - the compositions of the invention may comprise a softening agent such as clay and optionally also with flocculants and enzymes; optionally for softening through the wash.

### Processes of Making Compositions

The compositions of the present invention can be formulated into any suitable form and prepared by any process chosen by the formulator, non-limiting examples of which are described in Applicants' examples and in U.S. 4,990,280; U.S. 20030087791A1; U.S. 20030087790A1; U.S. 20050003983A1; U.S. 20040048764A1; U.S. 4,762,636; U.S. 6,291,412; U.S. 20050227891A1; EP 1070115A2; U.S. 5,879,584; U.S. 5,691,297; U.S. 5,574,005; U.S. 5,569,645; U.S. 5,565,422; U.S. 5,516,448; U.S. 5,489,392; U.S. 5,486,303 .

### Method of Use

The present invention includes a method for laundering a fabric. The method comprises the steps of contacting a fabric to be laundered with a said cleaning laundry solution comprising at least one embodiment of Applicants' cleaning composition, cleaning additive or mixture thereof. The fabric may comprise most any fabric capable of being laundered in normal consumer use conditions. The solution preferably has a pH of from about 8 to about 10.5. The compositions may be employed at concentrations of from about 500 ppm to about 15,000 ppm in solution. The water temperatures typically range from about 5 °C to about 90 °C. The water to fabric ratio is typically from about 1:1 to about 30:1.

### TEST METHOD 1

Fabric hueing agents are known to those skilled in the art and are described in the present specification. In one non-limiting aspect, suitable fabric hueing agents may be defined by the following test:
1) Fill two tergotometer pots with 800ml of Newcastle upon Tyne, UK, City Water (~12 grains per US gallon total hardness, supplied by Northumbrian Water, Pity Me, Durham, Co. Durham, UK).
2) Insert pots into tergotometer, with water temperature controlled at 30°C and agitation set at 40rpm for the duration of the experiment
3) Add 4.8g of IEC-B detergent (IEC 60456 Washing Machine Reference Base Detergent Type B), supplied by wfk, Brüggen-Bracht, Germany, to each pot.
4) After two minutes, add 2.0mg active colorant to the first pot.
5) After one minute, add 50g of flat cotton vest (supplied by Warwick Equest, Consett, County Durham, UK), cut into 5cm x 5cm swatches, to each pot.
6) After 10 minutes, drain the pots and re-fill with cold Newcastle upon Tyne City Water (16°C)
7) After 2 minutes rinsing, remove fabrics
8) Repeat steps 3-7 for a further three cycles using the same treatments
9) Collect and line dry the fabrics indoors for 12 hours
10) Analyse the swatches using a Hunter Miniscan spectrometer fitted with D65 illuminant and UVA cutting filter, to obtain Hunter a (red-green axis) and Hunter b (yellow-blue axis) values.
11) Average the Hunter a and Hunter b values for each set of fabrics. If the fabrics treated with colorant under assessment show an average difference in hue of greater than 0.2 units on either the a axis or b axis, it is deemed to be a fabric hueing agent for the purpose of the invention.

### EXAMPLES

Unless otherwise indicated, materials can be obtained from Aldrich, P.O. Box 2060, Milwaukee, WI 53201, USA.

### Examples 1-6

Granular laundry detergent compositions designed for handwashing or top-loading washing machines.

| | **1** | **2** | **3** | **4** | **5** | **6** |
|---|---|---|---|---|---|---|
| | (wt %) | (wt %) | (wt %) | (wt %) | (wt %) | (wt %) |
| Linear alkylbenzenesulfonate | 20 | 22 | 20 | 15 | 20 | 20 |
| C₁₂₋₁₄ Dimethylhydroxyethyl ammonium chloride | 0.7 | 1 | 1 | 0.6 | 0.0 | 0.7 |
| AE3S | 0.9 | 0.0 | 0.9 | 0.0 | 0.0 | 0.9 |
| AE7 | 0.0 | 0.5 | 0.0 | 1 | 3 | 1 |
| Sodium tripolyphosphate | 23 | 30 | 23 | 5 | 12 | 23 |
| Zeolite A | 0.0 | 0.0 | 1.2 | 0.0 | 10 | 0.0 |
| 1.6R Silicate (SiO₂:Na₂O at rat 1.6:1) | 7 | 7 | 7 | 7 | 7 | 7 |
| Sodium Carbonate | 15 | 14 | 15 | 18 | 15 | 15 |
| Polyacrylate MW 4500 | 1 | 0.0 | 1 | 1 | 1.5 | 1 |
| Carboxy Methyl Cellulose | 0.2 | 0.3 | 0.3 | 0.3 | 0.4 | 0.2 |
| Savinase® 32.89mg/g | 0.1 | 0.07 | 0.1 | 0.1 | 0.1 | 0.1 |
| Natalase® 8.65mg/g | 0.1 | 0.1 | 0.1 | 0.0 | 0.1 | 0.1 |
| Endoglucanase 15.6mg/g | 0.03 | 0.07 | 0.3 | 0.1 | 0.07 | 0.4 |
| Fluorescent Brightener 1 | 0.06 | 0.0 | 0.06 | 0.18 | 0.06 | 0.06 |
| Fluorescent Brightener 2 | 0.1 | 0.06 | 0.1 | 0.0 | 0.1 | 0.1 |
| Diethylenetriamine pentaacetic acid | 0.6 | 0.3 | 0.6 | 0.25 | 0.6 | 0.6 |
| MgSO₄ | 1 | 1 | 1 | 0.5 | 1 | 1 |
| Sodium Percarbonate | 0.0 | 5.2 | 0.1 | 0.0 | 0.0 | 0.0 |
| Sodium Perborate Monohydrate | 4.4 | 0.0 | 3.85 | 2.09 | .0.78 | 3.63 |
| NOBS | 1.9 | 0.0 | 1.66 | - | 0.33 | 0.75 |
| TAED | 0.58 | 1.2 | 0.51 | - | 0.015 | 0.28 |
| Sulphonated zinc phthalocyanine | 0.0030 | - | 0.0012 | 0.0030 | 0.0021 | - |
| S-ACMC | 0.1 | 0.06 | - | - | - | |
| Direct Violet 9 | - | - | 0.0003 | 0.0005 | 0.0003 | - |
| Ultramarine Blue | - | - | - | - | - | 0.2 |
| Sulfate/Moisture | Balance to 100% | Balance to 100% | Balance to 100% | Balance to 100% | Balance to 100% | Balance to 100% |

Any of the above compositions is used to launder fabrics at a concentration of 600 - 10000 ppm in water, with typical median conditions of 2500ppm, 25°C, and a 25:1 water:cloth ratio.

### Examples 7-10

Granular laundry detergent compositions designed for front-loading automatic washing machines.

| | **7** | **8** | **9** | **10** |
|---|---|---|---|---|
| | (wt%) | (wt%) | (wt%) | (wt%) |
| Linear alkylbenzenesulfonate | 8 | 7.1 | 7 | 6.5 |
| AE3S | 0 | 4.8 | 0 | 5.2 |
| Alkylsulfate | 1 | 0 | 1 | 0 |
| AE7 | 2.2 | 0 | 3.2 | 0 |
| C₁₀₋₁₂ Dimethyl hydroxyethylammoniurr chloride | 0.75 | 0.94 | 0.98 | 0.98 |
| Crystalline layered silicate (δ-Na₂Si₂O₅) | 4.1 | 0 | 4.8 | 0 |
| Zeolite A | 20 | 0 | 17 | 0 |
| Citric Acid | 3 | 5 | 3 | 4 |
| Sodium Carbonate | 15 | 20 | 14 | 20 |
| Silicate 2R (SiO₂:Na₂O at ratio 2:1) | 0.08 | 0 | 0.11 | 0 |
| Soil release agent | 0.75 | 0.72 | 0.71 | 0.72 |
| Acrylic Acid/Maleic Acid Copolymer | 1.1 | 3.7 | 1.0 | 3.7 |
| Carboxymethylcellulose | 0.15 | 1.4 | 0.2 | 1.4 |
| Protease (56.00mg active/g) | 0.37 | 0.4 | 0.4 | 0.4 |
| Termamyl® (21.55mg active/g) | 0.3 | 0.3 | 0.3 | 0.3 |
| Endoglucanase 15.6mg/g | 0.05 | 0.15 | 0.2 | 0.5 |
| Natalase® (8.65mg active/g) | 0.1 | 0.14 | 0.14 | 0.3 |
| TAED | 3.6 | 4.0 | 3.6 | 4.0 |
| Percarbonate | 13 | 13.2 | 13 | 13.2 |
| Na salt of Ethylenediamine-N,N' disuccinic acid, (S,S) isomer (EDDS) | 0.2 | 0.2 | 0.2 | 0.2 |
| Hydroxyethane di phosphonate (HEDP) | 0.2 | 0.2 | 0.2 | 0.2 |
| MgSO₄ | 0.42 | 0.42 | 0.42 | 0.42 |
| Perfume | 0.5 | 0.6 | 0.5 | 0.6 |
| Suds suppressor agglomerate | 0.05 | 0.1 | 0.05 | 0.1 |
| Soap | 0.45 | 0.45 | 0.45 | 0.45 |
| Sodium sulfate | 22 | 33 | 24 | 30 |
| Sulphonated zinc phthalocyanine (active) | 0.0007 | 0.0012 | 0.0007 | - |
| S-ACMC | 0.01 | 0.01 | - | 0.01 |
| Direct Violet 9 (active) | - | - | 0.0001 | 0.0001 |
| Water & Miscellaneous | Balance to 100% | Balance to 100% | Balance to 100% | Balance to 100% |

Any of the above compositions is used to launder fabrics at a concentration of 10,000 ppm in water, 20-90 °C, and a 5:1 water:cloth ratio. The typical pH is about 10.

### Examples 11-16

### Heavy Duty Liquid laundry detergent compositions

| | **11** (wt%)100% SNOW | **12** (wt%)100% SNOWBALL | **13** (wt%)75% SNOW | **14** (wt%)50% SNOWBALL | **15** (wt%)100% SNOW (nil polymer) | **16** (wt%)75% SNOW (Nil polymer) |
|---|---|---|---|---|---|---|
| AES C₁₂₋₁₅ alkyl ethoxy (1.8) sulfate | 11 | 10 | 4 | 6.32 | 6.0 | 8.2 |
| Linear alkyl benzene sulfonate | 4 | 0 | 8 | 3.3 | 4.0 | 3.0 |
| HSAS | 0 | 5.1 | 3 | 0 | 2 | 0 |
| Sodium formate | 1.6 | 0.09 | 1.2 | 0.04 | 1.6 | 1.2 |
| Sodium hydroxide | 2.3 | 3.8 | 1.7 | 1.9 | 2.3 | 1.7 |
| Monoethanolamine | 1.4 | 1.490 | 1.0 | 0.7 | 1.35 | 1.0 |
| Diethylene glycol | 5.5 | 0.0 | 4.1 | 0.0 | 5.500 | 4.1 |
| Nonionic | 0.4 | 0.6 | 0.3 | 0.3 | 2 | 0.3 |
| Chelant | 0.15 | 0.15 | 0.11 | 0.07 | 0.15 | 0.11 |
| Citric Acid | 2.5 | 3.96 | 1.88 | 1.98 | 2.5 | 1.88 |
| C₁₂₋₁₄ dimethyl Amine Oxide | 0.3 | 0.73 | 0.23 | 0.37 | 0.3 | 0.225 |
| C₁₂₋₁₈ Fatty Acid | 0.8 | 1.9 | 0.6 | 0.99 | 0.8 | 0.6 |
| Borax | 1.43 | 1.5 | 1.1 | 0.75 | 1.43 | 1.07 |
| Ethanol | 1.54 | 1.77 | 1.15 | 0.89 | 1.54 | 1.15 |
| Ethoxylated (EO₁₅) tetraethylene pentaimine¹ | 0.3 | 0.33 | 0.23 | 0.17 | 0.0 | 0.0 |
| Ethoxylated hexamethylene diamine² | 0.8 | 0.81 | 0.6 | 0.4 | 0.0 | 0.0 |
| 1,2-Propanediol | 0.0 | 6.6 | 0.0 | 3.3 | 0.0 | 0.0 |
| Liquanase®* | 36.4 | 36.4 | 27.3 | 18.2 | 36.4 | 27.3 |
| Mannaway®* | 1.1 | 1.1 | 0.8 | 0.6 | 1.1 | 0.8 |
| Natalase®* | 7.3 | 7.3 | 5.5 | 3.7 | 7.3 | 5.5 |
| Endoglucanase* | 10 | 3.2 | 1 | 3.2 | 2.4 | 3.2 |
| Liquitint® Violet CT (active) | 0.006 | 0.002 | - | - | - | 0.002 |
| S-ACMC | - | - | 0.01 | 0.05 | 0.01 | 0.02 |
| Water, perfume, dyes & other components | Balance | Balance | Balance | Balance | Balance | Balance |

### Raw Materials and Notes For Composition Examples 1-16

Linear alkylbenzenesulfonate having an average aliphatic carbon chain length C₁₁-C₁₂ supplied by Stepan, Northfield, Illinois, USA
C₁₂₋₁₄ Dimethylhydroxyethyl ammonium chloride, supplied by Clariant GmbH, Sulzbach, Germany
AE3S is C₁₂₋₁₅ alkyl ethoxy (3) sulfate supplied by Stepan, Northfield, Illinois, USA
AE7 is C₁₂₋₁₅ alcohol ethoxylate, with an average degree of ethoxylation of 7, supplied by Huntsman, Salt Lake City, Utah, USA
Sodium tripolyphosphate is supplied by Rhodia, Paris, France
Zeolite A was supplied by Industrial Zeolite (UK) Ltd, Grays, Essex, UK
1.6R Silicate was supplied by Koma, Nestemica, Czech Republic
Sodium Carbonate was supplied by Solvay, Houston, Texas, USA
Polyacrylate MW 4500 is supplied by BASF, Ludwigshafen, Germany
Carboxy Methyl Cellulose is Finnfix® BDA supplied by CPKelco, Arnhem, Netherlands Savinase®, Natalase®, Termamyl®, Mannaway® and Liquanase® supplied by Novozymes, Bagsvaerd, Denmark
Endoglucanase: Celluclean®, supplied by Novozymes, Bagsvaerd, Denmark
Fluorescent Brightener 1 is Tinopal® AMS, Fluorescent Brightener 2 is Tinopal® CBS-X, Sulphonated zinc phthalocyanine and Direct Violet 9 was Pergasol® Violet BN-Z all supplied by Ciba Specialty Chemicals, Basel, Switzerland
Diethylenetriamine pentacetic acid was supplied by Dow Chemical, Midland, Michigan, USA
Sodium percarbonate supplied by Solvay, Houston, Texas, USA
Sodium perborate was supplied by Degussa, Hanau, Germany
NOBS is sodium nonanoyloxybenzenesulfonate, supplied by Eastman, Batesville, Arkansas, USA
TAED is tetraacetylethylenediamine, supplied under the Peractive® brand name by Clariant GmbH, Sulzbach, Germany
S-ACMC is carboxymethylcellulose conjugated with C.I. Reactive Blue 19, sold by Megazyme, Wicklow, Ireland under the product name AZO-CM-CELLULOSE, product code S-ACMC.
Ultramarine Blue was supplied by Holliday Pigments, Kingston upon Hull, UK
Soil release agent is Repel-o-tex® PF, supplied by Rhodia, Paris, France
Acrylic Acid/Maleic Acid Copolymer is molecular weight 70,000 and acrylate:maleate ratio 70:30, supplied by BASF, Ludwigshafen, Germany
Protease described in patent application US 6312936B1 supplied by Genencor International, Palo Alto, California, USA
Na salt of Ethylenediamine-N,N'-disuccinic acid, (S,S) isomer (EDDS) was supplied by Octel, Ellesmere Port, UK
Hydroxyethane di phosphonate (HEDP) was supplied by Dow Chemical, Midland, Michigan, USA
Suds suppressor agglomerate was supplied by Dow Corning, Midland, Michigan, USA HSAS is mid-branched alkyl sulfate as disclosed in US 6,020,303 and US 6,060,443 C₁₂₋₁₄ dimethyl Amine Oxide was supplied by Procter & Gamble Chemicals, Cincinnati, Ohio, USA
Nonionic is preferably a C₁₂-C₁₃ ethoxylate, preferably with an av degree of ethoxylation of 9.
Liquitint® Violet CT was supplied by Milliken, Spartanburg, South Carolina, USA)
* Numbers quoted in mg enzyme/ 100g
¹ as described in US 4,597,898..
² available under the tradename LUTENSIT® from BASF and such as those described in WO 01/05874

### <First Sequence;protein/1;Bacillus sp.>

### <Second Sequence;protein/1;Bacillus sp. KSM-S237>

## Claims

1. A composition comprising a fabric hueing agent and a bacterial alkaline enzyme exhibiting endo-beta-1,4-glucanase activity (E.C. 3.2.1.4), and from 0.1% to 60% by weight of surfactant.

2. A composition according to claim 1 wherein enzyme is a bacterial polypeptide endogenous to a member of the genus *Bacillus*.

3. A composition according to claims 1-2 wherein the enzyme is a polypeptide containing (i) at least one family 17 carbohydrate binding module and/or (ii) at least one family 28 carbohydrate binding module.

4. A composition according to claims 1-3 wherein the enzyme comprises a polypeptide endogenous to one of the following *Bacillus* species selected from the group consisting of: AA349 (DSM 12648), KSM S237, 1139, KSM 64, KSM N131, KSM 635 (FERM BP 1485), KSM 534 (FERM BP 1508), KSM 53 (FERM BP 1509), KSM 577 (FERM BP 1510), KSM 521 (FERM BP 1507), KSM 580 (FERM BP 1511), KSM 588 (FERM BP 1513), KSM 597 (FERM BP 1514), KSM 522 (FERM BP 1512), KSM 3445 (FERM BP 1506), KSM 425 (FERM BP 1505), and mixtures thereof.

5. A composition according to claims 1-4 wherein the enzyme is selected from the group consisting of:
(i) the endoglucanase having the amino acid sequence of positions 1 to position 773 of SEQ ID NO:1;
(ii) an endoglucanase having a sequence of at least 90%, preferably 94%, more preferably 97% and even more preferably 99%, 100% identity to the amino acid sequence of position 1 to position 773 of SEQ ID NO:1; or a fragment thereof has endo-beta-1,4-glucanase activity, when identity is determined by GAP provided in the GCG program using a GAP creation penalty of 3.0 and GAP extension penalty of 0.1;
(iii) mixtures thereof.

6. A composition according to claims 1-4 wherein the enzyme is an alkaline endoglucanase variant obtained by substituting the amino acid residue of a cellulase having an amino acid sequence exhibiting at least 90%, preferably 95%, more preferably 98%, 100% identity with the amino acid sequence represented by SEQ. ID NO:2 at (a) position 10, (b) position 16, (c) position 22, (d) position 33, (e) position 39, (f) position 76, (g) position 109, (h) position 242, (i) position 263, (j) position 308, (k) position 462, (1) position 466, (m) position 468, (n) position 552, (o) position 564, and/or (p) position 608 in SEQ ID NO:2 and/or at a position corresponding thereto with another amino acid residue.

7. A composition according to claim 5 wherein the enzyme is **characterised by** at least one of the following substitutions:
(a) at position 10: glutamine, alanine, proline or methionine, preferably glutamine;
(b) at position 16: asparagine or arginine, preferably asparagine;
(c) at position 22: proline;
(d) at position 33: histidine;
(e) at position 39: alanine, threonine or tyrosine, preferably alanine;
(f) at position 76: histidine, methionine, valine, threonine or alanine, preferably histidine;
(g) at position 109: isoleucine, leucine, serine or valine, preferably isoleucine;
(h) at position 242: alanine, phenylalanine, valine, serine, aspartic acid, glutamic acid, leucine, isoleucine, tyrosine, threonine, methionine or glycine, preferably alanine, phenylalanine or serine;
(i) at position 263: isoleucine, leucine, proline or valine, preferably isoleucine;
(j) at position 308: alanine, serine, glycine or valine, preferably alanine;
(k) at position 462: threonine, leucine, phenylalanine or arginine, preferably threonine;
(l) at position 466: leucine, alanine or serine, preferably leucine;
(m) at position 468: alanine, aspartic acid, glycine or lysine, preferably alanine;
(n) at position 552: methionine;
(o) at position 564: valine, threonine or leucine, preferably valine; an/or
(p) at position 608: isoleucine or arginine, preferably isoleucine.

8. A composition according to claims 6 and 7 wherein the enzyme is selected from the group consisting of the following endoglucanase variants: Egl-237, Egl-1139, Egl-64, Egl-N131b and mixtures thereof.

9. A composition according to claims 1-4 wherein the enzyme is an alkaline cellulase K having the following physical and chemical properties:
(1) Activity: Having a Cx enzymatic activity of acting on carboxymethyl cellulose along with a weak C₁ enzymatic activity and a weak beta-glucoxidase activity;
(2) Specificity on Substrates: Acting on carboxymethyl cellulose(CMC), crystalline cellulose, Avicell, cellobiose, and p-nitrophenyl cellobioside(PNPC);
(3) Having a working pH in the range of 4 to 12 and an optimum pH in the range of 9 to 10;
(4) Having stable pH values of 4.5 to 10.5 and 6.8 to 10 when allowed to stand at 40°C for 10 minutes and 30 minutes, respectively;
(5) Working in a wide temperature range of from 10 to 65°C with an optimum temperature being recognized at about 40°C;
(6) Influences of chelating agents: The activity not impeded with ethylenediamine tetraacetic acid (EDTA), ethyleneglycol-bis-(β-aminoethylether) N,N,N',N"-tetraacetic acid (EGTA), N,N-bis(carboxymethyl)glycine (nitrilotriacetic acid) (NTA), sodium tripolyphosphate (STPP) and zeolite;
(7) Influences of surface active agents: Undergoing little inhibition of activity by means of surface active agents such as sodium linear alkylbenzenesulfonates (LAS), sodium alkylsulfates (AS), sodium polyoxyethylene alkylsulfates (ES), sodium alphaolefinsulfonates (AOS), sodium alpha-sulfonated aliphatic acid esters (alpha-SFE), sodium alkylsulfonates (SAS), polyoxyethylene secondary alkyl ethers, fatty acid salts (sodium salts), and dimethyldialkylammonium chloride;
(8) Having a strong resistance to proteinases; and
(9) Molecular weight (determined by gel chromatography): Having a maximum peak at 180,000 ±10,000.

10. A composition according to claim 9 wherein the alkaline cellulase K is obtained by isolation from a culture product of *Bacillus* sp KSM-635.

11. A composition according to claims 1-3 wherein the enzyme is selected from the group consisting of:
Alkaline Cellulase K-534 from KSM 534, FERM BP 1508,
Alkaline Cellulase K-539 from KSM 539, FERM BP 1509,
Alkaline Cellulase K-577 from KSM 577, FERM BP 1510,
Alkaline Cellulase K-521 from KSM 521, FERM BP 1507,
Alkaline Cellulase K-580 from KSM 580, FERM BP 1511,
Alkaline Cellulase K-588 from KSM 588, FERM BP 1513,
Alkaline Cellulase K-597 from KSM 597, FERM BP 1514,
Alkaline Cellulase K-522 from KSM 522, FERM BP 1512,
Alkaline Cellulase E-II from KSM 522, FERM BP 1512,
Alkaline Cellulase E-III from KSM 522, FERM BP 1512.
Alkaline Cellulase K-344 from KSM 344, FERM BP 1506,
Alkaline Cellulase K-425 from KSM 425, FERM BP 1505, and mixtures thereof.

12. A composition according to claims 1-3 wherein the enzyme is selected from the group consisting of endoglucanases derived from *Bacillus* species KSM-N, preferably is the alkaline endoglucanase Egl-546H derived from *Bacillus* sp. KSM-N546.

13. A composition according to any of the preceding claims wherein the bacterial alkaline enzyme exhibiting endo-beta-1,4-glucanase activity is comprised at a level of from 0.00005% to 0.15%, preferably from 0.0002% to 0.02%, or more preferably from 0.0005% to 0.01 % by weight of pure enzyme.

14. A composition according to any of the preceding claims wherein said fabric hueing agent is selected from the group consisting of dyes, dye-clay conjugates, and mixtures thereof.

15. A composition according the any of the preceding claims wherein said hueing agent is comprised at a level of from 0.00003% to 0.1%, preferably from 0.00008% to 0.05%, or more preferably from 0.0001 % to 0.04% by weight.

16. The composition of Claim 14-15 wherein said dyes are selected from the group consisting of small molecule dyes, polymeric dyes, and mixtures thereof, and said dye-clay conjugates are selected from the group consisting of dye clay conjugates comprising at least one cationic/basic dye and a smectite clay, and mixtures thereof.

17. The composition of Claims 14-16 wherein said small molecule dyes are selected from the group consisting of Direct Violet 9, Direct Violet 35, Direct Violet 48, Direct Violet 51, Direct Violet 66, Direct Blue 1, Direct Blue 71, Direct Blue 80, Direct Blue 279, Acid Red 17, Acid Red 73, Acid Red 88, Acid Red 150, Acid Violet 15, Acid Violet 17, Acid Violet 24, Acid Violet 43, Acid Violet 49, Acid Blue 15, Acid Blue 17, Acid Blue 25, Acid Blue 29, Acid Blue 40, Acid Blue 45, Acid Blue 75, Acid Blue 80, Acid Blue 83, Acid Blue 90 and Acid Blue 113, Acid Black 1, Basic Violet 1, Basic Violet 3, Basic Violet 4, Basic Violet 10, Basic Violet 35, Basic Blue 3, Basic Blue 16, Basic Blue 22, Basic Blue 47, Basic Blue 66, Basic Blue 75, Basic Blue 159 and mixtures thereof, said polymeric dyes are selected from the group consisting of polymers containing conjugated chromogens, polymers with chromogens co-polymerised into the backbone of the polymer and mixtures thereof, said dye-clay conjugates are selected from dye clay conjugates comprising a dye selected from the group consisting of C.I. Basic Yellow 1 through 108, C.I. Basic Orange 1 through 69, C.I. Basic Red 1 through 118, C.I. Basic Violet 1 through 51, C.I. Basic Blue 1 through 164, C.I. Basic Green 1 through 14, C.I. Basic Brown 1 through 23, CI Basic Black 1 through 11, and a clay selected from the group consisting of Montmorillonite clay, Hectorite clay, Saponite clay and mixtures thereof.

18. The composition of Claim 15 comprising a small molecule dye selected from the group consisting of Acid Violet 17, Acid Violet 43, Acid Red 73, Acid Red 88, Acid Red 150, Acid Blue 25, Acid Blue 29, Acid Blue 45, Acid Blue 113, Acid Black 1, Direct Blue 1, Direct Blue 71, Direct Violet 51, and mixtures thereof.

19. The composition of Claim 15 comprising a small molecule dye selected from the group consisting of Acid Violet 17, Direct Blue 71, Direct Violet 51, Direct Blue 1, Acid Red 88, Acid Red 150, Acid Blue 29, Acid Blue 113 or mixtures thereof.

20. The composition of Claims 1-13 said wherein said dye is selected from the group consisting of sulfonated zinc phthalocyanine, sulfonated aluminium phthalocyanines, xanthene dyes and mixtures thereof, preferably is xanthene dyes, more preferably is erythrosine (food red 14).

21. A composition according to claims 1-14 wherein the fabric hueing agent is a dye-photobleach conjugate

22. The composition of any of the preceding claims said composition comprising an adjunct material, preferably a lipase enzyme.

23. A composition according to Claim 1 wherein said fabric hueing agent comprises a fabric hueing agent that is a dye and/or a dye-clay conjugates that satisfies the requirements of Test Method 1 of the present specification.

24. A process of cleaning and/or treating a surface or fabric comprising the steps of optionally washing and/or rinsing said surface or fabric, contacting said surface or fabric with the composition of any of the preceding claims, then optionally washing and/or rinsing said surface or fabric.

## Patentansprüche

1. Zusammensetzung, umfassend ein Stofftönungsmittel und ein bakterielles alkalisches Enzym, das Endo-beta-1,4-Glucanase-Aktivität aufweist (E.C. 3.2.1.4), und von 0,1 Gew.-% bis 60 Gew.-% Tensid.

2. Zusammensetzung nach Anspruch 1, wobei das Enzym ein bakterielles Polypeptid ist, das für einen Vertreter der Gattung Bacillus endogen ist.

3. Zusammensetzung nach Ansprüchen 1-2, wobei das Enzym ein Polypeptid ist, das (i) mindestens ein Kohlenhydrat-Bindemodul der Familie 17 und/oder (ii) mindestens ein Kohlenhydrat-Bindemodul der Familie 28 enthält.

4. Zusammensetzung nach Ansprüchen 1-3, wobei das Enzym ein Polypeptid umfasst, das für eine der folgenden Bacillus-Arten endogen ist, die ausgewählt sind aus der Gruppe bestehend aus: AA349 (DSM 12648), KSM S237, 1139, KSM 64, KSM N131, KSM 635 (FERM BP 1485), KSM 534 (FERM BP 1508), KSM 53 (FERM BP 1509), KSM 577 (FERM BP 1510), KSM 521 (FERM BP 1507), KSM 580 (FERM BP 1511), KSM 588 (FERM BP 1513), KSM 597 (FERM BP 1514), KSM 522 (FERM BP 1512), KSM 3445 (FERM BP 1506), KSM 425 (FERM BP 1505) und Mischungen davon.

5. Zusammensetzung nach Ansprüchen 1-4, wobei das Enzym ausgewählt ist aus der Gruppe bestehend aus:
(i) der Endoglucanase mit der Aminosäure-Sequenz von Positionen 1 bis Position 773 von SEQ-ID Nr. 1;
(ii) einer Endoglucanase mit einer Sequenz von mindestens 90 %, vorzugsweise 94 %, mehr bevorzugt 97 % und noch mehr bevorzugt 99 %, 100 % Identität mit der Aminosäure-Sequenz von Position 1 bis Position 773 von SEQ-ID Nr. 1; oder ein Fragment davon hat Endo-beta-1,4-Glucanase-Aktivität, wenn die Identität mittels dem im GCG-Programm bereitgestellten GAP mit einer GAP Creation Penalty von 3,0 und einer GAP Extension Penalty von 0,1 bestimmt wird; (iii) Mischungen davon.

6. Zusammensetzung nach Ansprüchen 1-4, wobei das Enzym eine alkalische Endoglucanase-Variante ist, die durch Substitution des Aminosäurerests einer Cellulase mit einer Aminosäure-Sequenz, die mindestens 90 %, vorzugsweise 95 %, mehr bevorzugt 98 %, 100 % Identität mit der Aminosäure-Sequenz, die durch SEQ- ID Nr. 2 dargestellt ist, an (a) Position 10, (b) Position 16, (c) Position 22, (d) Position 33, (e) Position 39, (f) Position 76, (g) Position 109, (h) Position 242, (i) Position 263, (j) Position 308, (k) Position 462, (l) Position 466, (m) Position 468, (n) Position 552, (o) Position 564 und/oder (p) Position 608 in SEQ-ID Nr. 2 und/oder an einer dementsprechenden Position aufweist, durch einen anderen Aminosäurerest hergestellt wird.

7. Zusammensetzung nach Ansprüchen 5, wobei das Enzym durch mindestens eine der folgenden Substitutionen **gekennzeichnet** ist:
(a) an Position 10: Glutamin, Alanin, Prolin oder Methionin, vorzugsweise Glutamin;
(b) an Position 16: Asparagin oder Arginin, vorzugsweise Asparagin;
(c) an Position 22: Prolin;
(d) an Position 33: Histidin;
(e) an Position 39: Alanin, Threonin oder Tyrosin, vorzugsweise Alanin;
(f) an Position 76: Histidin, Methionin, Valin, Threonin oder Alanin, vorzugsweise Histidin;
(g) an Position 109: Isoleucin, Leucin, Serin oder Valin, vorzugsweise Isoleucin;
(h) an Position 242: Alanin, Phenylalanin, Valin, Serin, Asparaginsäure, Glutaminsäure, Leucin, Isoleucin, Tyrosin, Threonin, Methionin oder Glycin, vorzugsweise Alanin, Phenylalanin oder Serin;
(i) an Position 263: Isoleucin, Leucin, Prolin oder Valin, vorzugsweise Isoleucin;
(j) an Position 308: Alanin, Serin, Glycin oder Valin, vorzugsweise Alanin;
(k) an Position 462: Threonin, Leucin, Phenylalanin oder Arginin, vorzugsweise Threonin;
(l) an Position 466: Leucin, Alanin oder Serin, vorzugsweise Leucin;
(m) an Position 468: Alanin, Asparaginsäure, Glycin oder Lysin, vorzugsweise Alanin;
(n) an Position 552: Methionin;
(o) an Position 564: Valin, Threonin oder Leucin, vorzugsweise Valin; und/oder
(p) an Position 608: Isoleucin oder Arginin, vorzugsweise Isoleucin.

8. Zusammensetzung nach Ansprüchen 6 und 7, wobei das Enzym ausgewählt ist aus der Gruppe bestehend aus den folgenden Endoglucanase-Varianten: Egl-237, Egl-1139, Egl-64, Egl-N131b und Mischungen davon.

9. Zusammensetzung nach Ansprüchen 1-4, wobei das Enzym eine alkalische Cellulase K mit den folgenden physikalischen und chemischen Eigenschaften ist:
( 1 ) Aktivität: Hat eine Cx-Enzymaktivität des Einwirkens auf Carboxymethylcellulose sowie eine schwache C₁-Enzymaktivität und eine schwache beta-Glucoxidase-Aktivität;
(2) Spezifität auf Substraten: Wirkt auf Carboxymethylcellulose (CMC), kristalline Cellulose, Avicell, Cellobiose und p-Nitrophenylcellobiosid (PNPC);
(3) Hat einen Wirkungs-pH-Wert im Bereich von 4 bis 12 und einen optimalen pH-Wert im Bereich von 9 bis 10;
(4) Hat stabile pH-Werte von 4,5 bis 10,5 und 6,8 bis 10, wenn es bei 40 °C für 10 Minuten bzw. 30 Minuten stehen gelassen wird;
(5) Wirkt in einem breiten Temperaturbereich von 10 bis 65 °C, wobei eine optimale Temperatur bei ungefähr 40 °C erkannt wird;
(6) Einflüsse von Komplexbildnern: Die Aktivität wird nicht behindert von Ethylendiamintetraessigsäure (EDTA), Ethylenglycol-bis-(β-aminoethylether)-N,N,N',N"-tetraessigsäure (EGTA), N,N-Bis(carboxymethyl)glycin(nitrilotriessigsäure) (NTA), Natriumtripolyphosphat (STPP) und Zeolith;
(7) Einflüsse von oberflächenaktiven Mitteln: Erfährt wenig Hemmung der Aktivität durch oberflächenaktive Mittel wie lineare Natriumalkylbenzolsulfonate (LAS), Natriumalkylsulfate (AS), Natriumpolyoxyethylenalkylsulfate (ES), Natrium-alpha-olefinsulfonate (AOS), alphasulfonierte aliphatische Natrium-Säureester (alpha-SFE), Natriumalkylsulfonate (SAS), sekundäre Polyoxyethylenalkylether, Fettsäuresalze (Natriumsalze) und Dimethyldialkylammoniumchlorid;
(8) Hat eine starke Beständigkeit gegenüber Proteinasen; und
(9) Molekulargewicht (durch Gelchromatographie bestimmt): Höchster Peak bei 180.000 ± 10.000.

10. Zusammensetzung nach Ansprüchen 9, wobei die alkalische Cellulase K durch Isolation aus einem Kulturprodukt von Bacillus sp KSM-635 hergestellt wird.

11. Zusammensetzung nach Ansprüchen 1-3, wobei das Enzym ausgewählt ist aus der Gruppe bestehend aus:
Alkalischer Cellulase K-534 aus KSM 534, FERM BP 1508,
Alkalischer Cellulase K-539 aus KSM 539, FERM BP 1509,
Alkalischer Cellulase K-577 aus KSM 577, FERM BP 1510,
Alkalischer Cellulase K-521 aus KSM 521, FERM BP 1507,
Alkalischer Cellulase K-580 aus KSM 580, FERM BP 1511,
Alkalischer Cellulase K-588 aus KSM 588, FERM BP 1513,
Alkalischer Cellulase K-597 aus KSM 597, FERM BP 1514,
Alkalischer Cellulase K-522 aus KSM 522, FERM BP 1512,
Alkalischer Cellulase E-II aus KSM 522, FERM BP 1512,
Alkalischer Cellulase E-III aus KSM 522, FERM BP 1512,
Alkalischer Cellulase K-344 aus KSM 344, FERM BP 1506,
Alkalischer Cellulase K-425 aus KSM 425, FERM BP 1505 und Mischungen davon.

12. Zusammensetzung nach Ansprüchen 1-3, wobei das Enzym ausgewählt ist aus der Gruppe bestehend aus Endoglucanasen, die aus der Bacillus-Art KSM-N gewonnen werden, bevorzugt ist die alkalische Endoglucanase Egl-546H, gewonnen aus Bacillus sp. KSM-N546.

13. Zusammensetzung nach einem der vorstehenden Ansprüche, wobei das bakterielle alkalische Enzym, das Endo-beta-1,4-Glucanase-Aktivität aufweist, in einer Konzentration von 0,00005 Gew.-% bis 0,15 Gew.-%, vorzugsweise von 0,0002 Gew.-% bis 0,02 Gew.-% oder mehr bevorzugt von 0,0005 Gew.-% bis 0,01 Gew.-% des reinen Enzyms enthalten ist.

14. Zusammensetzung nach einem der vorstehenden Ansprüche, wobei das Stofftönungsmittel ausgewählt ist aus der Gruppe bestehend aus Farbstoffen, Farbstoff-Ton-Konjugaten und Mischungen davon.

15. Zusammensetzung nach einem der vorstehenden Ansprüche, wobei das Tönungsmittel in einer Konzentration von 0,00003 Gew.-% bis 0,1 Gew.-%, vorzugsweise von 0,00008 Gew.-% bis 0,05 Gew.-% oder mehr bevorzugt von 0,0001 Gew.-% bis 0,04 Gew.-% enthalten ist.

16. Zusammensetzung nach Ansprüchen 14-15, wobei die Farbstoffe ausgewählt sind aus der Gruppe bestehend aus kleinmolekularen Farbstoffen, polymeren Farbstoffen und Mischungen davon, und wobei die Farbstoff-Ton-Konjugate ausgewählt sind aus der Gruppe bestehend aus Farbstoff-Ton-Konjugaten, die mindestens einen kationischen/basischen Farbstoff und einen Smectit-Ton umfassen, und Mischungen davon.

17. Zusammensetzung nach Ansprüchen 14-16, wobei die kleinmolekularen Farbstoffe ausgewählt sind aus der Gruppe bestehend aus Direktviolett 9, Direktviolett 35, Direktviolett 48, Direktviolett 51, Direktviolett 66, Direktblau 1, Direktblau 71, Direktblau 80, Direktblau 279, Säurerot 17, Säurerot 73, Säurerot 88, Säurerot 150, Säureviolett 15, Säureviolett 17, Säureviolett 24, Säureviolett 43, Säureviolett 49, Säureblau 15, Säureblau 17, Säureblau 25, Säureblau 29, Säureblau 40, Säureblau 45, Säureblau 75, Säureblau 80, Säureblau 83, Säureblau 90 und Säureblau 113, Säureschwarz 1, Basisviolett 1, Basisviolett 3, Basisviolett 4, Basisviolett 10, Basisviolett 35, Basisblau 3, Basisblau 16, Basisblau 22, Basisblau 47, Basisblau 66, Basisblau 75, Basisblau 159 und Mischungen davon, die polymeren Farbstoffe ausgewählt sind aus der Gruppe bestehend aus Polymeren, die konjugierte Chromogene enthalten, Polymeren mit Chromogen, die in die Hauptkette des Polymers copolymerisiert sind, und Mischungen davon, die Farbstoff-Ton-Konjugate ausgewählt sind aus Farbstoff-Ton-Konjugaten, umfassend Farbstoff, ausgewählt aus der Gruppe bestehend aus C.I. Basisgelb 1 bis 108, C.I. Basisorange 1 bis 69, C.I. Basisrot 1 bis 118, C.I. Basisviolett 1 bis 51, C.I. Basisblau 1 bis 164, C.I. Basisgrün 1 bis 14, C.I. Basisbraun 1 bis 23, CI Basisschwarz 1 bis 11, und einen Ton, ausgewählt aus der Gruppe bestehend aus Montmorillonit-Ton, Hectorit-Ton, Saponit-Ton und Mischungen davon.

18. Zusammensetzung nach Anspruch 15, umfassend einen keinmolekularen Farbstoff, ausgewählt aus der Gruppe bestehend aus Säureviolett 17, Säureviolett 43, Säurerot 73, Säurerot 88, Säurerot 150, Säureblau 25, Säureblau 29, Säureblau 45, Säureblau 113, Säureschwarz 1, Direktblau 1, Direktblau 71, Direktviolett 51 und Mischungen davon.

19. Zusammensetzung nach Anspruch 15, umfassend einen kleinmolekularen Farbstoff, ausgewählt aus der Gruppe bestehend aus Säureviolett 17, Direktblau 71, Direktviolett 51, Direktblau 1, Säurerot 88, Säurerot 150, Säureblau 29, Säureblau 113 oder Mischungen davon.

20. Zusammensetzung nach Ansprchen 1-13, wobei der Farbstoff ausgewählt ist aus der Gruppe bestehend aus sulfoniertem Zinkphthalocyanin, sulfonierten Aluminiumphthalocyaninen, Xanthen-Farbstoffen und Mischungen davon, vorzugsweise Xanthen-Farbstoffe ist, mehr bevorzugt Erythrosin ist (Lebensmittelrot 14).

21. Zusammensetzung nach Ansprüchen 1-14, wobei das Stofftönungsmittel ein Farbstoff-Photobleichmittel-Konjugat ist.

22. Zusammensetzung nach einem der vorstehenden Ansprüche, wobei die Zusammensetzung ein Zusatzmaterial, vorzugsweise ein Lipase-Enzym umfasst.

23. Zusammensetzung nach Anspruch 1, wobei das Stofftönungsmittel ein Stofftönungsmittel umfasst, das ein Farbstoff und/oder Farbstoff-Ton-Konjugate ist, die die Anforderungen von Testverfahren 1 der vorliegenden Patentschrift erfüllen.

24. Verfahren zur Reinigung und/oder Behandlung einer Oberfläche oder eines Stoffes, umfassend die Schritte des optionalen Waschens und/oder Spülens der Oberfläche oder des Stoffes, des Inkontaktbringens der Oberfläche oder des Stoffes mit der Zusammensetzung nach einem der vorstehenden Ansprüche, des anschließenden optionalen Waschens und/oder Spülens der Oberfläche oder des Stoffes.

## Revendications

1. Composition comprenant un agent teintant des tissus et une enzyme alcaline bactérienne présentant une activité d'endo-bêta-1,4-glucanase (E.C. 3.2.1.4), et de 0,1 % à 60 % en poids d'agent tensioactif.

2. Composition selon la revendication 1, dans laquelle l'enzyme est un polypeptide bactérien endogène à un membre du genre *Bacillus.*

3. Composition selon les revendications 1 à 2, dans laquelle l'enzyme est un polypeptide contenant (i) au moins un module de liaison d'hydrate de carbone de famille 17 et/ou (ii) au moins un module de liaison d'hydrate de carbone de famille 28.

4. Composition selon les revendications 1 à 3, dans laquelle l'enzyme comprend un polypeptide endogène à une des espèces suivantes de *Bacillus* choisies dans le groupe constitué de : AA349 (DSM 12648), KSM S237, 1139, KSM 64, KSM N131, KSM 635 (FERM BP 1485), KSM 534 (FERM BP 1508), KSM 53 (FERM BP 1509), KSM 577 (FERM BP 1510), KSM 521 (FERM BP 1507), KSM 580 (FERM BP 1511), KSM 588 (FERM BP 1513), KSM 597 (FERM BP 1514), KSM 522 (FERM BP 1512), KSM 3445 (FERM BP 1506), KSM 425 (FERM BP 1505), et leurs mélanges.

5. Composition selon les revendications 1 à 4, dans laquelle l'enzyme est choisie dans le groupe constitué de :
(i) l'endoglucanase ayant la séquence d'acides aminés de la position 1 à la position 773 de SEQ ID No. : 1 ;
(ii) une endoglucanase ayant une séquence d'une identité d'au moins 90 %, de préférence 94 %, plus préférablement 97 % et encore plus préférablement 99 %, 100 % par rapport à la séquence d'acides aminés de la position 1 à la position 773 de SEQ ID No. : 1 ; ou un fragment de celle-ci a une activité d'endo-bêta-1,4-glucanase, lorsque l'identité est déterminée par le GAP (trou) fourni dans le programme GCG en utilisant une pénalité de création de GAP de 3,0 et une pénalité d'extension de GAP de 0,1 ; (iii) leurs mélanges.

6. Composition selon les revendications 1 à 4, dans laquelle l'enzyme est un variant d'endoglucanase alcalin obtenu en remplaçant le résidu d'acide aminé d'une cellulase ayant une séquence d'acides aminés présentant au moins 90 %, de préférence 95 %, plus préférablement 98 %, 100 % d'identité avec la séquence d'acides aminés représentée par SEQ. ID No. : 2 à (a) la position 10, (b) la position 16, (c) la position 22, (d) la position 33, (e) la position 39, (f) la position 76, (g) la position 109, (h) la position 242, (i) la position 263, (j) la position 308, (k) la position 462, (1) la position 466, (m) la position 468, (n) la position 552, (o) la position 564, et/ou (p) la position 608 dans SEQ ID No. : 2 et/ou à une position correspondant à celle-ci par un autre résidu d'acide aminé.

7. Composition selon la revendication 5, dans laquelle l'enzyme est **caractérisée par** au moins une des substitutions suivantes :
(a) à la position 10 : glutamine, alanine, proline ou méthionine, de préférence glutamine ;
(b) à la position 16 : asparagine ou arginine, de préférence asparagine ;
(c) à la position 22 : proline ;
(d) à la position 33 : histidine ;
(e) à la position 39 : alanine, thréonine ou tyrosine, de préférence alanine ;
(f) à la position 76 : histidine, méthionine, valine, thréonine ou alanine, de préférence histidine ;
(g) à la position 109 : isoleucine, leucine, sérine ou valine, de préférence isoleucine ;
(h) à la position 242 : alanine, phénylalanine, valine, sérine, acide aspartique, acide glutamique, leucine, isoleucine, tyrosine, thréonine, méthionine ou glycine, de préférence alanine, phénylalanine ou sérine ;
(i) à la position 263 : isoleucine, leucine, proline ou valine, de préférence isoleucine ;
(j) à la position 308 : alanine, sérine, glycine ou valine, de préférence alanine ;
(k) à la position 462 : thréonine, leucine, phénylalanine ou arginine, de préférence thréonine ;
(l) à la position 466 : leucine, alanine ou sérine, de préférence leucine ;
(m) à la position 468 : alanine, acide aspartique, glycine ou lysine, de préférence alanine ;
(n) à la position 552 : méthionine ;
(o) à la position 564 : valine, thréonine ou leucine, de préférence valine ; et/ou
(p) à la position 608 : isoleucine ou arginine, de préférence isoleucine.

8. Composition selon les revendications 6 et 7, dans laquelle l'enzyme est choisie dans le groupe constitué des variants d'endoglucanase suivants : Egl-237, Egl-1139, Egl-64, Egl-N131b et leurs mélanges.

9. Composition selon les revendications 1 à 4, dans laquelle l'enzyme est une cellulase alcaline K ayant les propriétés physiques et chimiques suivantes :
(1) Activité : ayant une activité enzymatique Cx pour agir sur la carboxyméthylcellulose en même temps qu'une faible activité enzymatique C1 et une faible activité de bêta-glucoxydase ;
(2) Spécificité sur les substrats : agissant sur la carboxyméthylcellulose (CMC), la cellulose cristalline, l'Avicell, la cellobiose, et le p-nitrophényl cellobioside (PNPC) ;
(3) Ayant un pH de travail dans la gamme de 4 à 12 et un pH optimal dans la gamme de 9 à 10 ;
(4) Ayant des valeurs de pH stables de 4,5 à 10,5 et 6,8 à 10 lorsqu'on laisse reposer à 40 °C pendant 10 minutes et 30 minutes, respectivement ;
(5) Fonctionnant dans un large intervalle de température allant de 10 à 65 °C avec une température optimale étant reconnue à environ 40 °C ;
(6) Influences des agents chélatants : l'activité n'est pas entravée avec l'acide éthylène-diamine tétra-acétique (EDTA), l'acide éthylèneglycol-bis-(13-aminoéthyléther) N,N,N',N"-tétra-acétique (EGTA), le N,N-bis(carboxyméthyl)glycine (acide nitrilotriacétique) (NTA), le tripolyphosphate de sodium (STPP) et une zéolite ;
(7) Influences des agents tensioactifs : subissant une petite inhibition d'activité au moyen d'agents tensioactifs tels que les sulfonates d'alkylbenzène linéaires de sodium (LAS), les alkylsulfates de sodium (AS), les polyoxyéthylène alkylsulfates de sodium (ES), les alpha-oléfine-sulfonates de sodium (AOS), les esters d'acide aliphatique alpha-sulfonés de sodium (alpha-SFE), les alkylsulfonates de sodium (SAS), les alkyléthers secondaires de polyoxyéthylène, les sels d'acide gras (sels de sodium), et le chlorure de diméthyldialkylammonium ;
(8) Ayant une forte résistance aux protéinases ; et
(9) Masse moléculaire (déterminé par chromatographie sur gel) : ayant un pic maximum à 180 000 ± 10 000.

10. Composition selon la revendication 9, dans laquelle la cellulase alcaline K est obtenue par isolement d'un produit de culture de *Bacillus* sp KSM-635.

11. Composition selon les revendications 1 à 3, dans laquelle l'enzyme est choisie dans le groupe constitué de :
Cellulase alcaline K-534 de KSM 534, FERM BP 1508,
Cellulase alcaline K-539 de KSM 539, FERM BP 1509,
Cellulase alcaline K-577 de KSM 577, FERM BP 1510,
Cellulase alcaline K-521 de KSM 521, FERM BP 1507,
Cellulase alcaline K-580 de KSM 580, FERM BP 1511,
Cellulase alcaline K-588 de KSM 588, FERM BP 1513,
Cellulase alcaline K-597 de KSM 597, FERM BP 1514,
Cellulase alcaline K-522 de KSM 522, FERM BP 1512,
Cellulase alcaline E-II de KSM 522, FERM BP 1512,
Cellulase alcaline E-III de KSM 522, FERM BP 1512,
Cellulase alcaline K-344 de KSM 344, FERM BP 1506,
Cellulase alcaline K-425 de KSM 425, FERM BP 1505, et leurs mélanges.

12. Composition selon les revendications 1 à 3, dans laquelle l'enzyme est choisie dans le groupe constitué d'endoglucanases dérivées des espèces de *Bacillus* KSM-N, de préférence est l'endoglucanase alcaline Egl-546H dérivée de *Bacillus* sp. KSM-N546.

13. Composition selon l'une quelconque des revendications précédentes, dans laquelle l'enzyme alcaline bactérienne présentant une activité d'endo-bêta-1,4-glucanase est comprise à un taux allant de 0,00005 % à 0,15 %, de préférence de 0,0002 % à 0,02 %, ou plus préférablement de 0,0005 % à 0,01 % en poids d'enzyme pure.

14. Composition selon l'une quelconque des revendications précédentes, dans laquelle ledit agent teintant des tissus est choisi dans le groupe constitué de teintures, conjugués teinture-argile, et leurs mélanges.

15. Composition selon l'une quelconque des revendications précédentes, dans laquelle ledit agent teintant est compris à un taux allant de 0,00003 % à 0,1 %, de préférence de 0,00008 % à 0,05 %, ou plus préférablement de 0,0001 % à 0,04 % en poids.

16. Composition selon les revendications 14 à 15, dans laquelle lesdites teintures sont choisies dans le groupe constitué de teintures à petites molécules, teintures polymères, et leurs mélanges, et lesdits conjugués teinture-argile sont choisis dans le groupe constitué de conjugués de teinture argile comprenant au moins une teinture cationique/basique et une argile smectite, et leurs mélanges.

17. Composition selon la revendication 14 à 16, dans laquelle lesdites teintures à petites molécules sont choisies dans le groupe constitué de Direct Violet 9, Direct Violet 35, Direct Violet 48, Direct Violet 51, Direct Violet 66, Direct Blue 1, Direct Blue 71, Direct Blue 80, Direct Blue 279, Acid Red 17, Acid Red 73, Acid Red 88, Acid Red 150, Acid Violet 15, Acid Violet 17, Acid Violet 24, Acid Violet 43, Acid Violet 49, Acid Blue 15, Acid Blue 17, Acid Blue 25, Acid Blue 29, Acid Blue 40, Acid Blue 45, Acid Blue 75, Acid Blue 80, Acid Blue 83, Acid Blue 90 and Acid Blue 113, Acid Black 1, Basic Violet 1, Basic Violet 3, Basic Violet 4, Basic Violet 10, Basic Violet 35, Basic Blue 3, Basic Blue 16, Basic Blue 22, Basic Blue 47, Basic Blue 66, Basic Blue 75, Basic Blue 159 et leurs mélanges, lesdites teintures polymères sont choisies dans le groupe constitué de polymères contenant des chromogènes conjugués, des polymères avec des chromogènes co-polymérisés dans le squelette du polymère et leurs mélanges, lesdits conjugués teinture-argile sont choisis parmi les conjugués de teinture argile comprenant une teinture choisie parmi le groupe constitué de C.I. Basic Yellow 1 à 108, C.I. Basic Orange 1 à 69, C.I. Basic Red 1 à 118, C.I. Basic Violet 1 à 51, C.I. Basic Blue 1 à 164, C.I. Basic Green 1 à 14, C.I. Basic Brown 1 à 23, CI Basic Black 1 à 11, et une argile choisie dans le groupe constitué de l'argile montmorillonitique, l'argile hectorite, l'argile saponite et leurs mélanges.

18. Composition selon la revendication 15, comprenant une teinture à petites molécules choisie dans le groupe constitué d'Acid Violet 17, Acid Violet 43, Acid Red 73, Acid Red 88, Acid Red 150, Acid Blue 25, Acid Blue 29, Acid Blue 45, Acid Blue 113, Acid Black 1, Direct Blue 1, Direct Blue 71, Direct Violet 51, et leurs mélanges.

19. Composition selon la revendication 15, comprenant une teinture à petites molécules choisie dans le groupe constitué d'Acid Violet 17, Direct Blue 71, Direct Violet 51, Direct Blue 1, Acid Red 88, Acid Red 150, Acid Blue 29, Acid Blue 113 ou leurs mélanges.

20. Composition selon les revendications 1 à 13, dans laquelle ladite teinture est choisie dans le groupe constitué de phtalocyanine de zinc sulfonatée, phtalocyanines d'aluminium sulfonées, teintures de xanthène et leurs mélanges, de préférence est des teintures de xanthène, plus préférablement est l'érythrosine (Food Red 14).

21. Composition selon les revendications 1 à 14, dans laquelle l'agent teintant des tissus est un conjugué teinture-agent de photoblanchiment

22. Composition selon l'une quelconque des revendications précédentes, ladite composition comprenant un matériau additif, de préférence une enzyme lipase.

23. Composition selon la revendication 1, dans laquelle ledit agent teintant des tissus comprend un agent teintant des tissus qui est une teinture et/ou un conjugué teinture-argile qui satisfait les exigences du Procédé de test 1 de la présente description.

24. Procédé de nettoyage et/ou de traitement d'une surface ou d'un tissu comprenant les étapes consistant à facultativement laver et/ou rincer ladite surface ou ledit tissu, mettre en contact ladite surface ou ledit tissu avec la composition selon l'une quelconque des revendications précédentes, puis à facultativement laver et/ou rincer ladite surface ou ledit tissu.


### SEQUENCE LISTING

<110> The Procter & Gamble Company
<120> Detergent compositions
<130> CM3097F
<160> 2
<170> PatentIn version 3.3
<210> 1
   <211> 773
   <212> PRT
   <213> Bacillus sp.
<400> 1
   Ala Glu Gly Asn Thr Arg Glu Asp Asn Phe Lys His Leu Leu Gly Asn
   1 5 10 15
   Asp Asn Val Lys Arg Pro Ser Glu Ala Gly Ala Leu Gln Leu Gln Glu
   20 25 30
   Val Asp Gly Gln Met Thr Leu Val Asp Gln His Gly Glu Lys Ile Gln
   35 40 45
   Leu Arg Gly Met Ser Thr His Gly Leu Gln Trp Phe Pro Glu Ile Leu
   50 55 60
   Asn Asp Asn Ala Tyr Lys Ala Leu Ala Asn Asp Trp Glu Ser Asn Met
   65 70 75 80
   Ile Arg Leu Ala Met Tyr Val Gly Glu Asn Gly Tyr Ala Ser Asn Pro
   85 90 95
   Glu Leu Ile Lys Ser Arg Val Ile Lys Gly Ile Asp Leu Ala Ile Glu
   100 105 110
   Asn Asp Met Tyr Val Ile Val Asp Trp His Val His Ala Pro Gly Asp
   115 120 125
   Pro Arg Asp Pro Val Tyr Ala Gly Ala Glu Asp Phe Phe Arg Asp Ile
   130 135 140
   Ala Ala Leu Tyr Pro Asn Asn Pro His Ile Ile Tyr Glu Leu Ala Asn
   145 150 155 160
   Glu Pro Ser Ser Asn Asn Asn Gly Gly Ala Gly Ile Pro Asn Asn Glu
   165 170 175
   Glu Gly Trp Asn Ala Val Lys Glu Tyr Ala Asp Pro Ile Val Glu Met
   180 185 190
   Leu Arg Asp Ser Gly Asn Ala Asp Asp Asn Ile Ile Ile Val Gly Ser
   195 200 205
   Pro Asn Trp Ser Gln Arg Pro Asp Leu Ala Ala Asp Asn Pro Ile Asn
   210 215 220
   Asp His His Thr Met Tyr Thr Val His Phe Tyr Thr Gly Ser His Ala
   225 230 235 240
   Ala Ser Thr Glu Ser Tyr Pro Pro Glu Thr Pro Asn Ser Glu Arg Gly
   245 250 255
   Asn Val Met Ser Asn Thr Arg Tyr Ala Leu Glu Asn Gly Val Ala Val
   260 265 270
   Phe Ala Thr Glu Trp Gly Thr Ser Gln Ala Asn Gly Asp Gly Gly Pro
   275 280 285
   Tyr Phe Asp Glu Ala Asp Val Trp Ile Glu Phe Leu Asn Glu Asn Asn
   290 295 300
   Ile Ser Trp Ala Asn Trp Ser Leu Thr Asn Lys Asn Glu Val Ser Gly
   305 310 315 320
   Ala Phe Thr Pro Phe Glu Leu Gly Lys Ser Asn Ala Thr Asn Leu Asp
   325 330 335
   Pro Gly Pro Asp His Val Trp Ala Pro Glu Glu Leu Ser Leu Ser Gly
   340 345 350
   Glu Tyr Val Arg Ala Arg Ile Lys Gly Val Asn Tyr Glu Pro Ile Asp
   355 360 365
   Arg Thr Lys Tyr Thr Lys Val Leu Trp Asp Phe Asn Asp Gly Thr Lys
   370 375 380
   Gln Gly Phe Gly Val Asn Ser Asp Ser Pro Asn Lys Glu Leu Ile Ala
   385 390 395 400
   Val Asp Asn Glu Asn Asn Thr Leu Lys Val Ser Gly Leu Asp Val Ser
   405 410 415
   Asn Asp Val Ser Asp Gly Asn Phe Trp Ala Asn Ala Arg Leu Ser Ala
   420 425 430
   Asp Gly Trp Gly Lys Ser Val Asp Ile Leu Gly Ala Glu Lys Leu Thr
   435 440 445
   Met Asp Val Ile Val Asp Glu Pro Thr Thr Val Ala Ile Ala Ala Ile
   450 455 460
   Pro Gln Ser Ser Lys Ser Gly Trp Ala Asn Pro Glu Arg Ala Val Arg
   465 470 475 480
   Val Asn Ala Glu Asp Phe Val Gln Gln Thr Asp Gly Lys Tyr Lys Ala
   485 490 495
   Gly Leu Thr Ile Thr Gly Glu Asp Ala Pro Asn Leu Lys Asn Ile Ala
   500 505 510
   Phe His Glu Glu Asp Asn Asn Met Asn Asn Ile Ile Leu Phe Val Gly
   515 520 525
   Thr Asp Ala Ala Asp Val Ile Tyr Leu Asp Asn Ile Lys Val Ile Gly
   530 535 540
   Thr Glu Val Glu Ile Pro Val Val His Asp Pro Lys Gly Glu Ala Val
   545 550 555 560
   Leu Pro Ser Val Phe Glu Asp Gly Thr Arg Gln Gly Trp Asp Trp Ala
   565 570 575
   Gly Glu Ser Gly Val Lys Thr Ala Leu Thr Ile Glu Glu Ala Asn Gly
   580 585 590
   Ser Asn Ala Leu Ser Trp Glu Phe Gly Tyr Pro Glu Val Lys Pro Ser
   595 600 605
   Asp Asn Trp Ala Thr Ala Pro Arg Leu Asp Phe Trp Lys Ser Asp Leu
   610 615 620
   Val Arg Gly Glu Asn Asp Tyr Val Ala Phe Asp Phe Tyr Leu Asp Pro
   625 630 635 640
   Val Arg Ala Thr Glu Gly Ala Met Asn Ile Asn Leu Val Phe Gln Pro
   645 650 655
   Pro Thr Asn Gly Tyr Trp Val Gln Ala Pro Lys Thr Tyr Thr Ile Asn
   660 665 670
   Phe Asp Glu Leu Glu Glu Ala Asn Gln Val Asn Gly Leu Tyr His Tyr
   675 680 685
   Glu Val Lys Ile Asn Val Arg Asp Ile Thr Asn Ile Gln Asp Asp Thr
   690 695 700
   Leu Leu Arg Asn Met Met Ile Ile Phe Ala Asp Val Glu Ser Asp Phe
   705 710 715 720
   Ala Gly Arg Val Phe Val Asp Asn Val Arg Phe Glu Gly Ala Ala Thr
   725 730 735
   Thr Glu Pro Val Glu Pro Glu Pro Val Asp Pro Gly Glu Glu Thr Pro
   740 745 750
   Pro Val Asp Glu Lys Glu Ala Lys Lys Glu Gln Lys Glu Ala Glu Lys
   755 760 765
   Glu Glu Lys Glu Glu
   770
<210> 2
   <211> 824
   <212> PRT
   <213> Bacillus sp.
<220>
   <223> Bacillus sp. KSM-S237
<400> 2
   Met Met Leu Arg Lys Lys Thr Lys Gln Leu Ile Ser Ser Ile Leu Ile
   1 5 10 15
   Leu Val Leu Leu Leu Ser Leu Phe Pro Ala Ala Leu Ala Ala Glu Gly
   20 25 30
   Asn Thr Arg Glu Asp Asn Phe Lys His Leu Leu Gly Asn Asp Asn Val
   35 40 45
   Lys Arg Pro Ser Glu Ala Gly Ala Leu Gln Leu Gln Glu Val Asp Gly
   50 55 60
   Gln Met Thr Leu Val Asp Gln His Gly Glu Lys Ile Gln Leu Arg Gly
   65 70 75 80
   Met Ser Thr His Gly Leu Gln Trp Phe Pro Glu Ile Leu Asn Asp Asn
   85 90 95
   Ala Tyr Lys Ala Leu Ser Asn Asp Trp Asp Ser Asn Met Ile Arg Leu
   100 105 110
   Ala Met Tyr Val Gly Glu Asn Gly Tyr Ala Thr Asn Pro Glu Leu Ile
   115 120 125
   Lys Gln Arg Val Ile Asp Gly Ile Glu Leu Ala Ile Glu Asn Asp Met
   130 135 140
   Tyr Val Ile Val Asp Trp His Val His Ala Pro Gly Asp Pro Arg Asp
   145 150 155 160
   Pro Val Tyr Ala Gly Ala Lys Asp Phe Phe Arg Glu Ile Ala Ala Leu
   165 170 175
   Tyr Pro Asn Asn Pro His Ile Ile Tyr Glu Leu Ala Asn Glu Pro Ser
   180 185 190
   Ser Asn Asn Asn Gly Gly Ala Gly Ile Pro Asn Asn Glu Glu Gly Trp
   195 200 205
   Lys Ala Val Lys Glu Tyr Ala Asp Pro Ile Val Glu Met Leu Arg Lys
   210 215 220
   Ser Gly Asn Ala Asp Asp Asn Ile Ile Ile Val Gly Ser Pro Asn Trp
   225 230 235 240
   Ser Gln Arg Pro Asp Leu Ala Ala Asp Asn Pro Ile Asp Asp His His
   245 250 255
   Thr Met Tyr Thr Val His Phe Tyr Thr Gly Ser His Ala Ala Ser Thr
   260 265 270
   Glu Ser Tyr Pro Ser Glu Thr Pro Asn Ser Glu Arg Gly Asn Val Met
   275 280 285
   Ser Asn Thr Arg Tyr Ala Leu Glu Asn Gly Val Ala Val Phe Ala Thr
   290 295 300
   Glu Trp Gly Thr Ser Gln Ala Ser Gly Asp Gly Gly Pro Tyr Phe Asp
   305 310 315 320
   Glu Ala Asp Val Trp Ile Glu Phe Leu Asn Glu Asn Asn Ile Ser Trp
   325 330 335
   Ala Asn Trp Ser Leu Thr Asn Lys Asn Glu Val Ser Gly Ala Phe Thr
   340 345 350
   Pro Phe Glu Leu Gly Lys Ser Asn Ala Thr Asn Leu Asp Pro Gly Pro
   355 360 365
   Asp His Val Trp Ala Pro Glu Glu Leu Ser Leu Ser Gly Glu Tyr Val
   370 375 380
   Arg Ala Arg Ile Lys Gly Val Asn Tyr Glu Pro Ile Asp Arg Thr Lys
   385 390 395 400
   Tyr Thr Lys Val Leu Trp Asp Phe Asn Asp Gly Thr Lys Gln Gly Phe
   405 410 415
   Gly Val Asn Ser Asp Ser Pro Asn Lys Glu Leu Ile Ala Val Asp Asn
   420 425 430
   Glu Asn Asn Thr Leu Lys Val Ser Gly Leu Asp Val Ser Asn Asp Val
   435 440 445
   Ser Asp Gly Asn Phe Trp Ala Asn Ala Arg Leu Ser Ala Asn Gly Trp
   450 455 460
   Gly Lys Ser Val Asp Ile Leu Gly Ala Glu Lys Leu Thr Met Asp Val
   465                 470                 475                 480
   Ile Val Asp Glu Pro Thr Thr Val Ala Ile Ala Ala Ile Pro Gln Ser
                   485                 490                 495
   Ser Lys Ser Gly Trp Ala Asn Pro Glu Arg Ala Val Arg Val Asn Ala
               500                 505                 510
   Glu Asp Phe Val Gln Gln Thr Asp Gly Lys Tyr Lys Ala Gly Leu Thr
           515                 520                 525
   Ile Thr Gly Glu Asp Ala Pro Asn Leu Lys Asn Ile Ala Phe His Glu
       530                 535                 540
   Glu Asp Asn Asn Met Asn Asn Ile Ile Leu Phe Val Gly Thr Asp Ala
   545                 550                 555                 560
   Ala Asp Val Ile Tyr Leu Asp Asn Ile Lys Val Ile Gly Thr Glu Val
                   565                 570                 575
   Glu Ile Pro Val Val His Asp Pro Lys Gly Glu Ala Val Leu Pro Ser
               580                 585                 590
   Val Phe Glu Asp Gly Thr Arg Gln Gly Trp Asp Trp Ala Gly Glu Ser
           595                 600                 605
   Gly Val Lys Thr Ala Leu Thr Ile Glu Glu Ala Asn Gly Ser Asn Ala
       610                 615                 620
   Leu Ser Trp Glu Phe Gly Tyr Pro Glu Val Lys Pro Ser Asp Asn Trp
   625                 630                 635                 640
   Ala Thr Ala Pro Arg Leu Asp Phe Trp Lys Ser Asp Leu Val Arg Gly
                   645                 650                 655
   Glu Asn Asp Tyr Val Ala Phe Asp Phe Tyr Leu Asp Pro Val Arg Ala
               660                 665                 670
   Thr Glu Gly Ala Met Asn Ile Asn Leu Val Phe Gln Pro Pro Thr Asn
           675                 680                 685
   Gly Tyr Trp Val Gln Ala Pro Lys Thr Tyr Thr Ile Asn Phe Asp Glu
       690                 695                 700
   Leu Glu Glu Ala Asn Gln Val Asn Gly Leu Tyr His Tyr Glu Val Lys
   705                 710                 715                 720
   Ile Asn Val Arg Asp Ile Thr Asn Ile Gln Asp Asp Thr Leu Leu Arg
                   725                 730                 735
   Asn Met Met Ile Ile Phe Ala Asp Val Glu Ser Asp Phe Ala Gly Arg
               740                 745                 750
   Val Phe Val Asp Asn Val Arg Phe Glu Gly Ala Ala Thr Thr Glu Pro
           755                 760                 765
   Val Glu Pro Glu Pro Val Asp Pro Gly Glu Glu Thr Pro Pro Val Asp
       770                 775                 780
   Glu Lys Glu Ala Lys Lys Glu Gln Lys Glu Ala Glu Lys Glu Glu Lys
   785                 790                 795                 800
   Glu Ala Val Lys Glu Glu Lys Lys Glu Ala Lys Glu Glu Lys Lys Ala
                   805                 810                 815
   Val Lys Asn Glu Ala Lys Lys Lys
               820